(19) Europäisches Patentamt
European Patent Office
Office européen des brevets

(11) Veröffentlichungsnummer: **0 129 186**
**B1**

(12) **EUROPÄISCHE PATENTSCHRIFT**

(45) Veröffentlichungstag der Patentschrift: **16.08.90**

(21) Anmeldenummer: **84106684.8**

(22) Anmeldetag: **12.06.84**

(51) Int. Cl.$^5$: **C 07 D 233/60,**
C 07 D 249/08, A 01 N 43/50,
A 01 N 43/64

# (54) Azolderivate.

(30) Priorität: **14.06.83 DE 3321422**

(43) Veröffentlichungstag der Anmeldung:
**27.12.84 Patentblatt 84/52**

(45) Bekanntmachung des Hinweises auf die Patenterteilung:
**16.08.90 Patentblatt 90/33**

(84) Benannte Vertragsstaaten:
**AT BE CH DE FR GB IT LI NL SE**

(56) Entgegenhaltungen:
**EP-A-0 047 405**
**EP-A-0 057 357**
**EP-A-0 163 895**
**DE-A-2 638 470**
**FR-A-2 379 250**

(73) Patentinhaber: **BASF Aktiengesellschaft**
**Carl-Bosch-Strasse 38**
**D-6700 Ludwigshafen (DE)**

(72) Erfinder: **Buschmann, Ernst, Dr.**
**Georg-Ludwig-Krebs-Strasse 10**
**D-6700 Ludwigshafen (DE)**
Erfinder: **Sauter, Hubert, Dr.**
**Neckarpromenade**
**D-6800 Mannheim 1 (DE)**
Erfinder: **Tuerk, Wolfgang, Dr.**
**Pranckhstrasse 28**
**D-6700 Ludwigshafen (DE)**
Erfinder: **Ammermann, Eberhard, Dr.**
**Sachsenstrasse 3**
**D-6700 Ludwigshafen (DE)**
Erfinder: **Pommer, Ernst-Heinrich, Dr.**
**Berliner Platz 7**
**D-6703 Limburgerhof (DE)**

**EP 0 129 186 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft neue, wertvolle Azolverbindungen, Verfahren zu ihrer Herstellung, diese Verbindungen enthaltende fungizide Mittel und ihre Verwendung als Fungizide.

Es ist bekannt, daß Triazolderivate, zum Beispiel das 2,2-Dimethyl-4-(1,2,4-triazol-1-yl)-5-phenylpentanon-3 (DE—OS 26 38 470) eine gute fungizide Wirksamkeit zeigen. Die Wirkung ist jedoch bei niedrigen Aufwandmengen und Anwendungskonzentrationen nicht immer befriedigend. Darüber hinaus ist die fungitoxische Wirkung oft mit einer hohen Phytotoxizität verbunden, so daß in den für die Bekämpfung von Pilzen im Pflanzenschutz, beispielsweise bei der Bekämpfung von Rostpilzen notwendigen Konzentrationen auch die Kulturpflanzen geschädigt werden. Aus diesen Gründen sind sie für den Gebrauch als Pflanzenschutzmittel zur Bekämpfung von Pilzen nicht immer und nicht bei allen Pflanzenarten geeignet. Aus FR—A—2 379 250 sind Azolverbindungen bekannt, die einen gegebenenfalls substituierten Benzylrest enthalten und das Pflanzenwachstum beeinflussen.

Es wurden nun neue Verbindungen gefunden, die eine sehr hohe fungitoxische Wirkung bei ausgezeichneter Pflanzenverträglichkeit aufweisen.

Gegenstand der Erfindung sind neue Azolverbindungen der Formel I

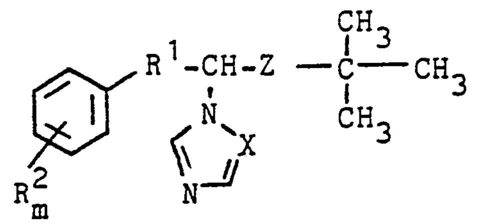

I,

in der bedeuten

$R^1$ eine Alkylenkette mit 3 bis 10 Kohlenstoffatomen, die durch 1 bis 3 $C_1$- bis $C_3$-Alkylreste substituiert sein kann,

$R^2$ Wasserstoff, Halogen, $C_1$- bis $C_4$-Alkyl, $C_1$- bis $C_4$-Halogenalkyl, $C_1$- bis $C_4$-Alkoxy, $C_2$- bis $C_5$-Alkenoyl, $C_1$- bis $C_4$-Alkylthio, $C_1$- bis $C_4$-Alkylsulfinyl, $C_1$- bis $C_4$-Alkylsulfonyl, Trifluormethyl, Cyano, Phenoxy oder Benzyloxy,

m 1, 2, 3,

X CH, N

Z Carbonyl oder die Gruppe

$>CR^3OR^4$,

in der $R^3$ Wasserstoff, $C_1$- bis $C_4$-Alkyl oder $C_2$- bis $C_4$-Alkenyl und $R^4$ Wasserstoff, $C_1$- bis $C_4$-Alkyl, $C_2$- bis $C_4$-Alkenyl, $C_2$- bis $C_4$-Alkinyl, $C_1$- bis $C_4$-Alkenoyl oder Benzyl bedeutet, sowie deren für Pflanzen verträgliche Säureadditionssalze und Metallkomplexe.

Die erfindungsgemäßen Verbindungen der Formel I fallen bei ihrer Herstellung als Enantiomerengemische bzw. Diastereomerengemische an, die in Üblicher Weise, z.B. durch Kristallisation oder Chromatographie, in die einzelnen Komponenten getrennt und als Diastereomere A oder B oder (3S, 4S; 3R, 4R) oder (3S, 4R, 3R, 4S) bezeichnet werden können. Für die Anwendung der erfindungsgemäßen Verbindungen als Fungizide ist jedoch eine Trennung der Enantiomeren oder Diastereomeren normalerweise nicht erforderlich.

Der Arylrest kann beispielsweise folgendermäßen durch $R^2$ substituiert sein, wobei die 1 bis 3 Reste $R^2$ identisch oder unterschiedlich sein können:

| | |
|---|---|
| Wasserstoff | 3-Methyl-, |
| 2-Fluor-, | 4-Methyl-, |
| 4-Fluor-, | 3-tert.-Butyl-, |
| 2-Chlor-, | 4-tert.-Butyl-, |
| 3-Chlor-, | 2-Methoxy-, |
| 4-Chlor-, | 3-Methoxy-, |
| 4-Brom-, | 4-Methoxy-, |
| 2,4-Dichlor-, | 3,5-Dimethoxy-, |
| 2,4,6-Trichlor-, | 3-n-Butoxy-, |
| 3,5-Dichlor-, | 4-n-Butoxy-, |
| 2-Chlor-4-phenyl-, | 2-Methoxy-4-methyl-, |
| 2-Methyl-4-chlor-, | 3-Trifluormethyl-, |
| | 4-Trifluormethyl-. |

$R^1$ bedeutet beispielsweise Propylen, Butylen, Pentylen, Hexylen, Heptylen, Oktylen, Nonylen, Decylen, 2-Methylpropylen, 3-Ethylpropylen, 3-Butylpropylen, 3-Methylpentylen, 3,5-Dimethylpentylen, 2-Methylpentylen,

$R^3$ bedeutet beispielsweise Wasserstoff, Methyl, Ethyl, n-Propyl, Isopropyl, n-Butyl, Vinyl, Allyl,

$R^4$ bedeutet beispielsweise Wasserstoff, Methyl, Ethyl, n-Propyl, Prop-2-en-yl (1), Prop-2-inyl (1), n-Butyl, Acetyl, Propionyl, Butyryl oder Isobutyryl.

Geeignete Säureadditionssalze sind beispielsweise Bromide, Sulfate, Nitrate, Phosphate, Oxalate, Dodecylsulfonate. Die Wirksamkeit der Salze geht auf das Kation zurück, so daß die Wahl des Anions, sieht man von der Pflanzenverträglichkeit ab, beliebig ist.

Geeignete Metallkomplexe sind Verbindungen der Formel IX

$$Me[(I)_y]Q_x \qquad IX,$$

in der I die oben angegebene Bedeutung hat,

Me ein Äquivalent eines Metallions, z.B. Kupfer, Zink, Zinn, Mangan, Eisen, Cobalt oder Nickel,

Q ein Äquivalent des Anions einer anorganischen Säure, z.B. Salzsäure, Schwefelsäure, Phosphorsäure oder Bromwasserstoffsäure, bedeutet und x und Y für die zum Ausgleich der Wertigkeiten erforderlichen Zahlen stehen.

Gegenstand der Erfindung sind weiter Verfahren zur Herstellung der Azolverbindungen der Formel I, gemäß Anspruch 1, dadurch gekennzeichnet, daß man

a) Ketone der Formel II

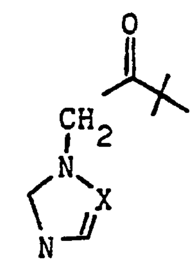

II,

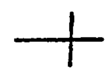 = tert.-Butyl

in der X die angegebenen Bedeutungen hat, oder deren Alkalienolate mit einem ω-Arylalkylhalogenid der Formel III

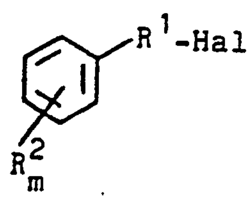

III,

in der $R^2$ und m die in Anspruch 1 angegebenen Bedeutungen haben und Hal für Chlor, Brom oder Iod steht, gegebenenfalls in Gegenwart eines Lösungs- oder Verdünnungsmittels und/oder anorganischer oder organischer Basen bei Temperaturen zwischen 0 und 200°C umsetzt oder daß man

b) eine Verbindung der allgemeinen Formel IV

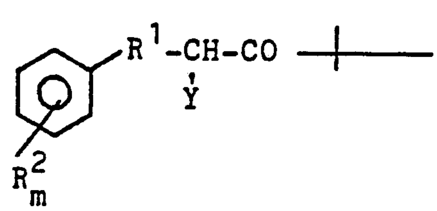

IV,

in der $R^1$, $R^2$ und m die vorgenannten Bedeutungen haben und Y Chlor oder Brom bedeutet, mit Imidazol oder 1,2,4-Triazol gegebenenfalls in Gegenwart eines Lösungs- oder Verdünnungsmittels und/oder anorganischer Basen bei Temperaturen zwischen 0 und 100°C umsetzt

und gewünschtenfalls

c) die so erhaltene Verbindung der Formel I, in der Z für eine CO-Gruppe steht, durch Einwirkung eines komplexen Hybrids oder durch Einwirkung von Wasserstoff in Gegenwart eines Hydrierungskatalysators, gegebenenfalls in Gegenwart eines Lösungs- oder Verdünnungsmittels, bei Temperaturen zwischen 0 und

100°C zum sekundären Alkohol der Formel I, in der Z für eine CHOH-Gruppe steht, reduziert oder

d) mit einer Grignardverbindung der Formel VII

$$R^3—MgHal \qquad VII$$

in der $R^3$ für $C_1$- bis $C_4$-Alkyl oder $C_2$- bis $C_4$-Alkenyl steht und in der Hal Chlor, Brom oder Iod bedeutet, gegebenenfalls in Gegenwart eines Lösungs- oder Verdünnungsmittels und gegebenenfalls in Gegenwart eines Magnesium- oder Tetraalkylammoniumhalogenids bei Temperaturen zwischen 0 und 100°C umsetzt und die so entstandenen Alkoholate zu den tertiären Alkoholen hydrolysiert und die so nach c) erhaltenen sekundären oder nach d) erhaltenen tertiären Alkohole — falls gewünscht — mit einem $C_1$- bis $C_4$-Alkanoylchlorid oder einem $C_1$- bis $C_4$-Alkanoylanhydrid gegebenenfalls in Gegenwart eines Lösungs- oder Verdünnungsmittels und/oder einer anorganischen oder organischen Base und/oder eines Acylierungskatalysators bei Temperaturen zwischen 0 und 100°C oder deren Alkali- oder deren quartären Ammoniumsalze mit einem Alkylierungsmittel der Formel VIII

$$L—R^4 \qquad VIII$$

in der $R^4$ $C_1$- bis $C_4$-Alkyl, $C_2$- bis $C_4$-Alkenyl oder $C_2$- bis $C_4$-Alkinyl bedeutet und L für eine nucleophil verdrängbare Abgangsgruppe steht, gegebenenfalls in Gegenwart eines Lösungs- oder Verdünnungsmittels und/oder anorganischer oder organischer Basen und/oder eines Reaktionsbeschleunigers bei Temperaturen zwischen 0 und 100°C umsetzt oder daß man

e) ein Grignardreagenz der allgemeinen Formel

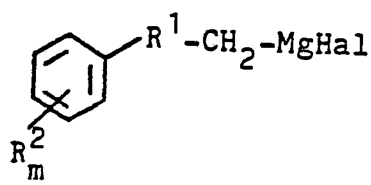

V,

in der $R^1$, $R^2$ und m die in Anspruch 1 genannten Bedeutungen haben und Hal für Chlor, Brom oder Iod steht, mit tert.-Butylcyanid nach an sich bekannten Methoden zu dem entsprechenden Keton VI

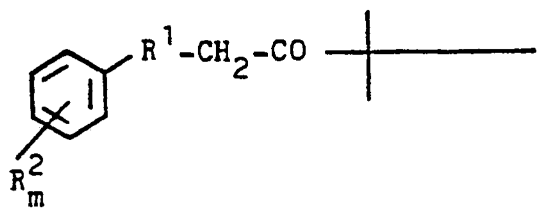

VI

umsetzt, dieses zu Verbindungen der Formel IV halogeniert und das so erhaltene Halogenid mit Imidazol oder 1,2,4-Triazol gegebenenfalls in Gegenwart eines Lösungs- oder Verdünnungsmittels und/oder anorganischer oder organischer Basen bei Temperaturen zwischen 0 und 100°C umsetzt und gewünschtenfalls weiter nach c) oder d) verfährt,

worauf man gewünschtenfalls die jeweils erhaltenen Verbindungen der Formel I in ihre für Pflanzen verträgliche Säureadditionssalze oder Metallkomplexe überführt.

Die Vorprodukte können auf folgende Weise erhalten werden:

Man kondensiert Pinakolon mit einem entsprechenden Aldehyd der Formel X

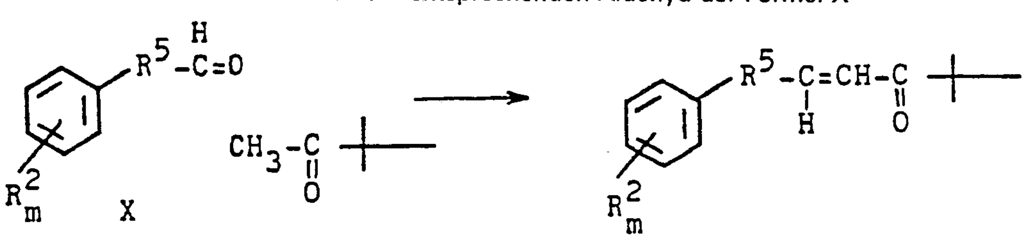

X

in der $R^2$ und m die unter Anspruch 1 angegebenen Bedeutungen haben und $R^5 = R^1$ minus 1C ist, nach an sich bekannten Verfahren, gegebenenfalls in Gegenwart eines Lösungs- oder Verdünnungsmittels und/oder anorganischer oder organischer Basen bei Temperaturen zwischen 0 und 100°C. Besonders soll

erwähnt sein ein organisches Lösungsmittel wie beispielsweise Toluol in Gegenwart eines Katalysators wie beispielsweise Piperidinacetat.

Man reduziert die Doppelbindung durch katalytische Hydrierung mit Platin, Palladium oder Praseodym-Katalysatoren auf inerten Trägern. Man hydriert mit oder ohne inertes Lösungsmittel bei Drücken von 2—100 bar, bis keine Wasserstoffaufnahme mehr erfolgt.

Man halogeniert das Keton der allgemeinen Formel VI

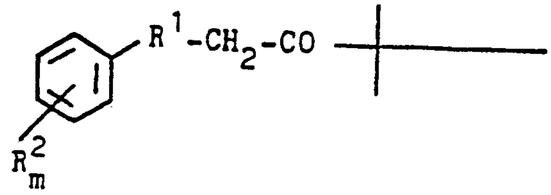

VI.

nach an sich bekannten Methoden wie z.B. mit Sulfurylchlorid nach D. P. Wymann und P. R. Kaufmann, J. Org. Chem. 29 (1964) 1956 oder mit Brom in Formamid nach H. Bredereck et. al. Chem. Ber. *93* (1960) 2083 zu den Zwischenprodukten IV, die gegebenenfalls in Gegenwart eines Lösungs- oder Verdünnungsmittels und/oder anorganischer oder organischer Basen bei Temperaturen zwischen 0 und 100°C mit Imidazol oder 1,2,4-Triazol zu den Wirkstoffen der Formel I umgesetzt werden können.

Das Verfahren zur Herstellung der Ketone der Formel I, (d.h., in denen für Z eine CO-Gruppe steht) ist in folgenden drei Synthesewegen aufgezeigt:

Weg a) besteht darin, daß man bekannte Ketone der Formel II (DE—OS 26 38 470) oder deren Alalienolate mit ω-Arylalkylhalogeniden der Formel III, gegebenenfalls in Gegenwart einer Base und/oder eines Lösungs- oder Verdünnungsmitteis, zu den erfindungsgemäßen Ketonen der Formel I alkyliert.

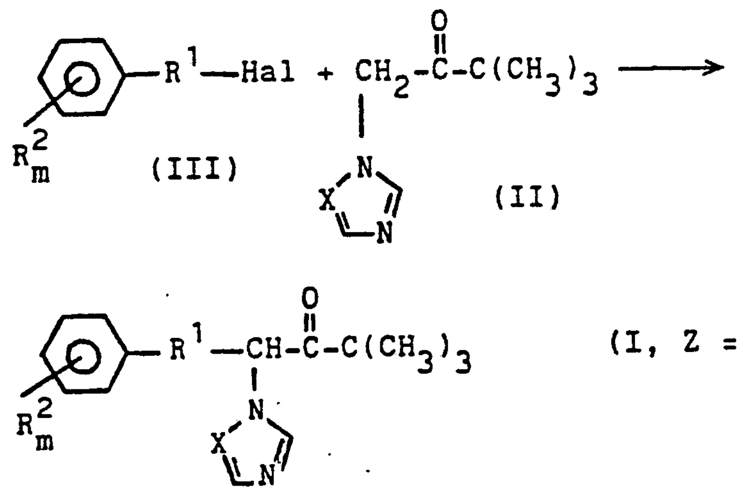

(III) (II)

(I, Z = CO)

Hierzu können die Ketone II zunächst zu den Alkalienolaten metalliert werden, indem man sie vorzugsweise in Gegenwart eines polaren aprotischen Lösungsmittels wie Dimethylformamid, Acetonitril oder Tetrahydrofuran mit 0,8 bis 1,2 Äquivalenten, bevorzugt 1,0 Äquivalenten, eines Metallierungsreagens wie Natriumhydrid, Lithiumdiisopropylamid oder n-Butyllithium bei 0 bis 200°C, vorzugsweise bei 10 bis 50°C umsetzt. Nach anschließender Zugabe von 0,8 bis 2,0, bevorzugt 1,0 Äquivalenten des jeweiligen ω-Arylalkylhalogenids der Formel III erhält man bei Reaktionstemperatur zwischen 0 und 200°C, bevorzugt bei 30 bis 150°C die Ketone der Formel I.

Eine Variante dieses Verfahrens besteht darin, daß man die Ketone in Gegenwart von 0,8 bis 1,2, bevorzugt 1,0 Äquivalenten einer Base wie z.B. Kalium-tert.-butoxid, Natriummethoxid oder Kaliumhydroxid mit den ω-Arylalkylhalogeniden umsetzt, wobei man zweckmäßigerweise in Gegenwart eines Lösungs- oder Verdünnungsmittels bei Temperaturen zwischen 0 und 200°C, bevorzugt bei 30 bis 150°, arbeitet.

Als Lösungsmittel oder Verdünnungsmittel kommen hier wiederum dipolare aprotische Lösungsmittel in Betracht, aber auch Alkohole wie Methanol oder tert.-Butanol.

Weg b) besteht darin, daß nach an sich bekannten Methoden ein Zwischenprodukt der allgemeinen Formel IV, in der $R^1$, $R^2$ und m die vorgenannten Bedeutungen haben und Y Chlor oder Brom bedeuten, gegebenenfalls in Gegenwart eines Lösungs- oder Verdünnungsmittels und/oder anorganischer oder organischer Basen bei Temperaturen zwischen 0 und 100°C mit Imidazol oder 1,2,4-Triazol umgesetzt wird.

Die Ketone der allgemeinen Formel VI werden zum Beispiel, wie oben angegeben, durch Kondensation einer Aldehydkomponente der allgemeinen Formel X mit Pinakolon und anschließender katalytischer Hydrierung mit Pd-Katalysator und Wasserstoff erhalten.

Die Aldehydkomponente wird mit Pinakolon gegebenenfalls in Gegenwart eines Lösungs- oder Verdünnungsmittels und gegebenenfalls unter Zusatz einer anorganischen oder organischen Base und/ oder Säure bei Temperaturen zwischen −10 und 150°C gegebenenfalls unter azeotropischer Auskreisung des Reaktionswassers kondensiert. Zu den bevorzugten Lösungs- und Verdünnungsmitteln gehören Wasser, Alkohole wie Methanol, Ethanol, Isopropanol und tert.-Butanol, Kohlenwasserstoff wie n-Hexan, n-Heptan, n- und iso-Octan, Cyclohexan, Tetrahydronaphthalin, Decahydronaphthalin, Toluol, Xylol, Cumol; Amide wie Dimethylformamid, Dimethylacetamid, N-Methylpyrrolidon und Ether wie Diethylether, Methyl-tert.-butylether, Dioxan, Tetrahydrofuran oder entsprechende Gemische.

Geeignete Basen und Säuren, die gegebenenfalls in katalytischen bis zu stöchiometrischen Mengen verwendet werden können, sind beispielsweise Alkali- und Erdalkalihydroxide, wie Natrium-, Kalium-, Calcium- und Bariumhydroxid, Alkoxide wie Natrium- und Kaliummethoxid, -ethoxid, -isopropoxid und -tert.-butoxid, Acetate wie Natrium- oder Kaliumacetat, Amine wie Pyrrolidin, Piperidin, Triethylamin, N,N-Dimethylcyclohexylamin, ferner anorganische Säuren wie Salzsäure, Phosphorsäure und Schwefelsäure oder organische Säuren wie Ameisensäure, Essigsäure, p-Toluolsulfonsäure oder Trifluormethylsulfonsäure.

Die Umsetzungen werden im allgemeinen bei Temperaturen zwischen 20 und 150°C, drucklos oder unter Druck, kontinuierlich oder diskontinuierlich durchgeführt.

Zur katalytischen Hydrierung verwendet man Platin oder Palladiumkatalysatoren auf inerten Trägern und hydriert bei Drucken von 2—100 bar bis keine Wasserstoffaufnahme mehr erfolgt.

Die anschließende Halogenierung der Ketone VI zu den Zwischenprodukten IV kann in einem organischen Lösungsmittel wie Ether, Tetrachlorkohlenstoff, Tetrahydrofuran, Eisessig (mit Brom) oder Formamid mit Brom bei einer Temperatur von 0 bis 100°C erfolgen.

Weg c): Als weiteres Verfahren zur Herstellung der erfindungsgemäßen Verbindungen der allgemeinen Formel I über die Ketone VI kann die Reaktion eines Grignardreagenz der allgemeinen Formel V

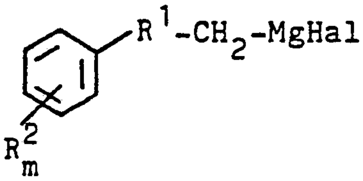

V,

mit tert.-Butylcyanid angesehen werden.

Als Lösungsmittel kommen bevorzugt Ether in Frage wie Diethylether, Di-n-propylether, Tetrahydrofuran.

Die Reaktionstemperaturen lassen sich je nach Lösungsmittel zwischen 0 bis 100°C variieren, bevorzugt sind Temperaturen zwischen 0 und 60°C. Die primär entstanden Magnesylkomplexe werden, sodann durch Hydrolyse mit Wasser oder verdünnten wäßrigen Säuren wie Salzsäure, Schwefelsäure oder Essigsäure, oder bevorzugt mit wäßriger Ammonchloridlösung in die entsprechenden Ketone übergeführt und diese nach Entfernen der wäßrigen Phase, falls gewünscht, in üblicher Weise durch Extraktion, Umkristallisation oder Chromatographie gereinigt.

Die anschließende Bromierung und weitere Umsetzung mit Imidazol oder 1,2,4-Triazol erfolgt in vorgenannter Weise zu den entsprechenden Wirkstoffen I (Z = O).

Das Verfahren zur Herstellung der sekundären Alkohole der Formel I, in der Z die Gruppe

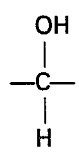

darstellt, besteht darin, daß man die Ketone der Formel I, in denen Z eine CO-Gruppe ist, einer Reduktion unterwirft, z.B. durch Umsetzung mit einer Alkyl-Grignard-Verbindung mit Wasserstoff in beta-Stellung des Alkylrestes, wie n-Propyl-magnesiumbromid in Gegenwart eines geeigneten aprotischen Lösungsmittels, wie Ether oder Tetrahydrofuran, bei Temperaturen von 0°C bis zum Siedepunkt des Lösungsmittels und anschließende Hydrolyse des sekundären Magnesiumalkoholats in üblicher Weise oder z.B. durch Einwirkung von komplexen Hydriden, bevorzugt Natriumborhydrid in Gegenwart eines polaren Lösungsmittels, z.B. eines Alkohols, bevorzugt Methanol oder Ethanol bei Temperaturen zwischen 0 und 100°C und anschließender Hydrolyse mit wäßrigen Basen oder Säuren oder durch Einwirkung von

Wasserstoff in Gegenwart eines Hydrierungskatalysators wie Platin oder Raney-Nickel und in Gegenwart eines polaren Lösungsmittels wie Methanol, Ethanol oder Ethylacetat bei Temperaturen zwischen 20 und 100°C und Drücken von 1 bis 100 bar:

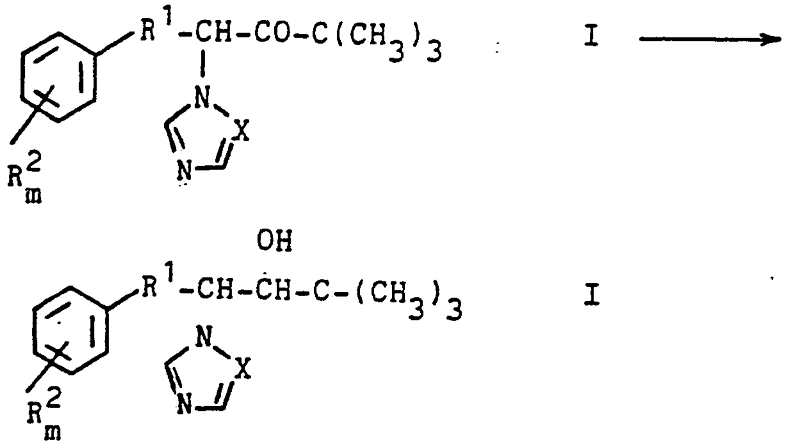

Das Verfahren zur Herstellung der tertiären Alkohole der Formel I (Z =

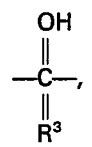

worin $R^3$ für $C_1$- bis $C_4$-Alkyl, nicht aber für Wasserstoff steht), besteht darin, daß man die Ketone der Formel I (Z = CO) mit 0,8 bis 1,2 Äquivalenten einer Grignardverbindung der Formel VII

$$R^3\text{-MgHal} \qquad \text{VII,}$$

in der $R^3$ $C_1$- bis $C_4$-Alkyl und Hal Chlor, Brom oder Iod bedeutet, vorzugsweise in Abwesenheit eines Lösungsmittels und gegebenenfalls in Anwesenheit eines ausbeutesteigerndem Salzes umsetzt:

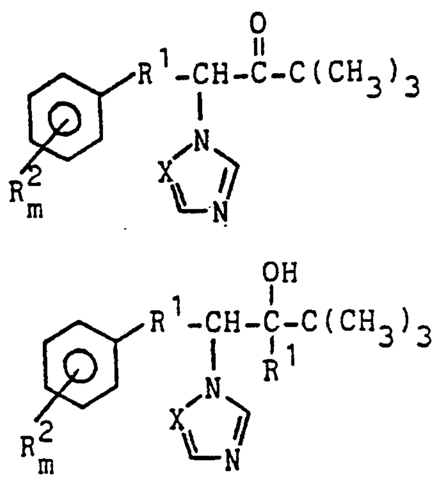

I

I

Als Lösungsmittel kommen bevorzugt Ether in Frage, wie Diethylether, Di-n-propylether, Tetrahydrofuran oder Anisol, ferner tertiäre Amine wie N,N-Diethylanilin sowie Phosphorsäure-tris(dimethylamid); gegebenenfalls kann man die Reaktion auch im Gemisch dieser Lösungsmittel mit alphatischen oder aromatischen Kohlenwasserstoffen wie n-Hexan oder Toluol durchführen. Als ausbeutesteigernde Salze, die die üblichen Nebenreaktionen unterdrücken, kommen insbesondere wasserfreie Magnesiumhalogenide wie wasserfreies Magnesiumbromid oder wasserfreie Tetraalkylammoniumhalogenide wie z.B. Tetra-n-butylammoniumchlorid in Frage. Die Reaktionstemperaturen lassen sich je nach Lösungsmittel zwischen 0 und 100°C variieren, bevorzugt sind

Temperaturen zwischen 0 und 60°C. Die hierbei primär entstandenen Magnesiumalkoholate werden sodann durch Hydrolyse mit verdünnten wäßrigen Säuren, wie Salzsäure, Schwefelsäure oder bevorzugt Essigsäure oder besonders bevorzugt mit wäßriger Ammoniumchloridlösung in die Alkohole übergeführt und diese nach Entfernen der wäßrigen Phase, falls gewünscht, in üblicher Weise durch Extraktion, Umkristallisieren oder Chromatographie gereinigt.

Das Verfahren zur Herstellung der Ester der Formel I

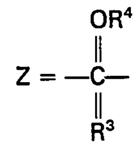

in der $R^4$ Acetyl, Propionyl, n-Butyryl oder Isobutyryl bedeutet), besteht darin, daß man die sekundären oder tertiären Alkohole der Formel I

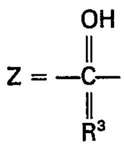

mit den entsprechenden Säurechloriden oder Säureanhydriden in Gegenwart eines säurebindenden Mittels und gegebenenfalls in Gegenwart eines aprotischen Lösungsoder Verdünnungsmittels sowie bezorzugt in Gegenwart eines Acylierungskatalysators bei Temperaturen zwischen 0 und 100°C, vorzugsweise zwischen 10 und 50°C umsetzt. Als säurebindende Mittel können anorganische Basen wie Natriumamid oder besonders bevorzugt Pyridin in mindestens äquivalenten Mengen eingesetzt werden. Als Acylierungskatalysatoren verwendet man zweckmäßigerweise Imidazol oder 4-Dimethylaminopyridin in Mengenanteilen von 0,01 bis 0,4 Äquivalenten, falls nicht bereits Pyridin anwesend ist. Als Lösungsmittel können Kohlenwasserstoffe wie Cyclohexan oder Toluol, Ether wie Diethylether, Tetrahydrofuran oder Dioxan, Ketone wie Aceton oder Diethylketon oder auch überschüssige säurebindende Amine wie Triethylamin oder Pyridin eingesetzt werden.

Das Verfahren zur Herstellung der Ether der Formel I

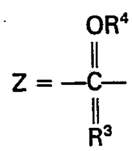

in der $R^4$ $C_1$- bis $C_4$-Alkyl,

$C_2$- bis $C_4$-Alkenyl oder
$C_2$- bis $C_4$-Alkinyl bedeutet, besteht darin, daß man die sekundären oder tertiären Alkohole der Formel I

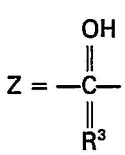

oder ihr Alkali- oder quartären Ammoniumsalze mit einem Alkylierungsmittel der Formel VIII

$$L—R^4 \qquad VIII$$

gegebenenfalls in Gegenwart eines Lösungs- oder Verdünnungsmittels gegebenenfalls in Gegenwart anorganischer oder organischer Basen und/oder eines Reaktionsbeschleunigers zwischen 0 und 100°C umsetzt.

Für die im obigen Verfahren erwähnten nucleophil verdrängbaren Abgangsgruppen L seien beispielsweise genannt: Halogen, vorzugsweise Chlor, Brom oder Iod; Alkylsulfat, vorzugsweise Methylsulfat; gegebenenfalls substituierte Alkylsulfonyloxyreste, vorzugsweise Methansulfonyloxy- oder

Trifluormethansulfonyloxyreste; oder Arylsulfonyloxyreste, vorzugsweise der Tosylatrest.

Geeignete anorganische oder organische Basen, die gegebenenfalls auch als säurebindende Mittel in die Reaktion eingesetzt werden können, sind beispielsweise Alkali- und Erdalkalihydroxide wie Natriumhydroxid, Kaliumhydroxid, Calciumhydroxid, Alkalicarbonate wie Kalium- oder Natriumcarbonat, Alkalihydride wie Natriumhydrid, Alkali- oder Erdalkalialkoholate wie Natriummethylat, Magnesiummethylat oder Natriumisopropylat oder tertiäre Amine wie Trimethylamin, Triethylamin, N,N-Dimethylanilin, N,N-Dimethylcyclohexylamin, N-Methylpiperidin oder Pyridin. Es können aber auch andere übliche Basen verwendet werden.

Mit geeigneten Basen wie z.B. Alkalihydrid wie Natriumhydrid oder Lithiumalkylen wie Butyllithium oder mit Alkali- oder Erdalkalialkoholaten wie Natriummethylat können die Alkohole der Formel I

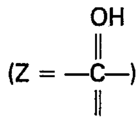

auch in einer vorgeschalteten Umsetzung zunächst in ihre Alkoholat-Salze überführt werden und dann als solche zur Reaktion gebracht werden.

Zu den bevorzugten Lösungs- bzw. Verdünnungsmitteln gehören Halogenkohlenwasserstoffe wie beispielsweise Methylenchlorid, Chloroform, 1,2-Dichlorethan, Chlorbenzol; aliphatische oder aromatische Kohlenwasserstoffe wie Cyclohexan, Petrolether, Benzol, Toluol oder Xylole, Ester wie Essigsäureethylester, Amide wie Dimethylformamid, Nitrile wie Acetonitril, Sulfoxide wie Dimethylsulfoxid, Ketone wie Aceton oder Methylethylketon, Ether wie Diethylether, Tetrahydrofuran oder Dioxan oder entsprechende Gemische.

Als Reaktionsbeschleuniger kommen vorzugsweise Metallhalogenide wie Kaliumiodid, Kronenether, quartäre Ammoniumverbindungen wie Tetrabutylammoniumiodid oder Säuren oder Kombinationen dieser Reaktionsbeschleuniger in Frage.

Die erfindungsgemäßen Umsetzungen werden im allgemeinen bei Temperaturen zwischen 0 und 100°C in einem Zeitraum von 1 bis 60 Stunden durchgeführt, drucklos oder unter Druck, kontinuierlich oder diskontinuierlich.

Zur Isolierung der erfindungsgemäßen Verbindungen wird nach üblichen Methoden vorgegangen. Im allgemeinen bedürfen die anfallenden Produkte keiner weiteren Reinigung, sie können aber nach bekannten Methoden wie Umkristallisation, Extraktion, Destillation oder Chromatographie weiter gereinigt werden.

In den Azolverbindungen der Formel I ist das Azolyl-substituierte Kohlenstoffatom chiral. Je nach Abwesenheit bzw. Anwesenheit weiterer Chiralitätszentren in der Alkylenkette $R^1$ und/oder in der Gruppierung Z fallen die Produkte der Formel I normalerweise als Enantiomeren- bzw. Diastereomerengemische an, die mit üblichen Methoden in die einzelnen Isomeren getrennt werden können. Die einzelnen Isomeren zeigen unterschiedliche biologische Aktivität; sie sind ebenso wie die Isomerengemische Gegenstand der vorliegenden Erfindung, d.h. die reinen Diastereomeren und die reinen Enantiomeren sind ebenso wie deren Gemische Bestandteil der Erfindung. Für den Einsatz als Fungizide genügt die Verwendung der Gemische, wenn auch in gewissen Fällen die Verwendung von reinen Isomeren eine Reduzierung der Aufwandmenge ermöglicht.

Falls gewünscht, können die erfindungsgemäßen Verbindungen der Formel I auch in Salze mit anorganischen oder organischen Säuren übergeführt werden, wie beispielsweise in Salze der Salzsäure, Bromwasserstoffsäure, Salpetersäure, Oxalsäure, Essigsäure, Schwefelsäure, Phosphorsäure oder Dodecylbenzolsulfonsäure. Die Wirksamkeit der Salze geht auf das Kation zurück, so daß die Wahl des Anions beliebig ist.

Ferner lassen sich die Verbindungen der Formel I nach bekannten Methoden in Metallkomplexe überführen. Das kann durch Umsetzung dieser Verbindungen mit geeigneten Metallsalzen wie beispielsweise Kupfer(II)-chlorid, Zink(II)-chlorid, Eisen(III)-chlorid, Kupfer(II)-nitrat, Mangan(II)-chlorid oder Nickel(II)-bromid erfolgen.

Die Herstellung der erfindungsgemäßen Verbindungen der Formel I wird durch folgende Beispiele erläutert:

Herstellung der Ausgangsmaterialien

Vorschrift 1A

Herstellung von 2,2-Dimethyl-7-phenyl-hepta-dien(4,6)on-(3)

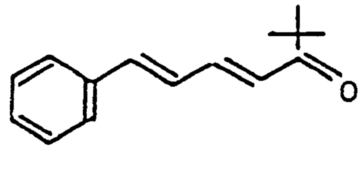

229 g (1,735 Mol) Zimtaldehyd werden in 100 ml Ethanol mit 347 g (3,47 Mol) Pinakolon versetzt. In dieses Reaktionsgemisch wird innerhalb von 30 Min. 3,6 g (0,09 mol) NaOH ≙ 36 g 10 %iges NaOH zugetropft.

Nach Rühren bei 35°C während 3 Stunden wird das Reaktionsgemisch eingeengt und mit Methylenchlorid und Wasser aufgenommen. Die organische Phase wird über Natriumsulfat getrocknet und eingeengt. Eine anschließende Destillation im Vakuum ergibt eine bei 132 bis 150°C (0,2 mm Hg) siedende Fraktion von 90 g 2,2-Dimethyl-7-phenyl-hepta-dien(4,6)-on-(3)

$^{1}H$—NMR (100 MHz, $CDCl_3$)
1,2 ppm (s, 9H)
6,6—6,9 ppm (m)
7,2—7,6 ppm (s, breit)

Vorschrift 1B

Herstellung von 2,2-Dimethyl-7-phenyl-heptan-on(3)

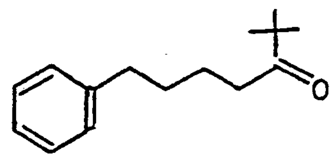

90 g (0,4 Mol) 2,2-Dimethyl-7-phenylheptadien(4,6)on-(3) werden an 20 g Kontakt (5% Praseodym; 0,5% Pd auf $Al_2O_3$) in einem Autoklaven in 1000 ml Methanol bei 30 bar Wasserstoffdruck und einer Temperatur von 100°C bis zur Druckkonstanz hydriert.

Nach beendeter Reaktion wird das Reaktionsgemisch abgekühlt und eingeengt. Der entstandene Festkörper wird abgesaugt und getrocknet.

Auf diese Weise erhält man 64 g (≙ 70,8%) 2,2-Dimethyl-7-phenylheptan-on(3)

$^{1}H$—NMR (100 MHz, $CDCl_3$)
1,1 ppm (s)
1,6 ppm (m)
2,4—2,7 ppm (m)
7,2 ppm (s, breit)

Vorschrift 1C

Herstellung von 2,2-Dimethyl-4-bromo-7-phenylheptanon-(3)

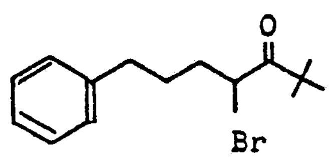

In einer Lösung von 64 g (0,29 Mol) 2,2-Dimethyl-7-phenylheptanon-(3) in 250 ml absolutem Ether wird innerhalb von 2 Stunden bei 30°C 52,7 g (0,29 Mol) Brom in 100 ml Ether zugetropft. Das Reaktionsgemisch wird nach Rühren über Nacht bei der Raumtemperatur mit 200 ml Wasser versetzt. Nach Abtrennen der organischen Phase wird diese mit Natriumcarbonat gewaschen und über Natriumsulfat gewaschen. Nach Abdestillieren des Lösungsmittels erhält man 100 g Rohprodukt.

$^{1}H$—NMR (60 MHz, $CDCl_3$)
1,2 (s, breit)
1,8—2,1 (m, breit)
2,5—2,8 (t, breit)
3,5 (E)
5,6 (t, breit)
7,2 (s, breit)

Herstellung des Endproduktes

Beispiel 1

2,2-Dimethyl-4-(1,2,4-triazol-1-yl)-7-phenyl-heptanon-(3)

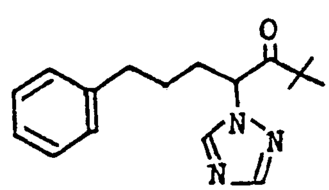

Eine unter Stickstoff gerührte Suspension von 51,1 g (0,74 Mol) Triazol und 39,2 g (0,37 Mol) Natriumcarbonat in 500 ml Ethanol wird auf Rückflußtemperatur erhitzt und mit 110 g (0,37 Mol) 2,2-Dimethyl-4-brom-7-phenyl-heptanon-(3) in 300 ml Ethanol versetzt. Nach 3 Stunden Rühren wird das Lösungsmittel abdestilliert und der Rückstand mit Wasser und Methylenchlorid extrahiert. Die organische Phase wird nach Waschen und Trocknen mit Natriumsulfat eingeengt und fraktioniert destilliert.

Die bei 150 bis 154°C (0,3 torr) gesammelte Fraktion wird als 2,2-Dimethyl-4-(1,2,4-Triazol-1-yl)-7-heptanon-(3) identifiziert (Wirkstoff Beispiel Nr. 1).

$^1H$—NMR (100 MHz, $CDCl_3$)
1,1 (s)
1,4—2,2 (m, breit)
2,6 (t, breit)
5,5 (t)
7,2 (m)
7,9 und 8,3 (s)

Beispiel 2

2,2-Dimethyl-4-(1,2,4-triazol-1-yl-7-phenyl-heptanol-(3)

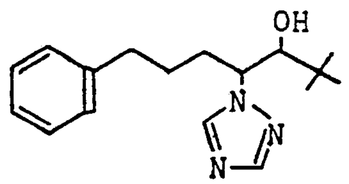

Zu einer Lösung von 40 g (0,14 Mol) 2,2-Dimethyl-4-(1,2,4-triazol-(1)-7-phenylheptanon(3) in 1000 ml Methanol werden zwischen 5 bis 10°C 3,4 g (0,09 Mol) Natriumborhydrid portionsweise zugegeben. Nach zweistündigem Rühren bei Raumtemperatur wird das Reaktionsgemisch eingeengt und 0,5 Stunden mit Methylenchlorid und 10 %iger Kalilauge gerührt. Die organische Schicht wird abgetrennt, dreimal mit 100 ml Wasser gewaschen, über Natriumsulfat getrocknet und eingeengt.

Durch Destillation erhält man bei 162 bis 175°C (0,2 torr) 27 g 2,2-Dimethyl-4-(1,2,4-Triazol-1-yl)-7-phenylheptanol-(3).

$^1H$—NMR (80 MHz, $CDCl_3$) (Wirkstoff Beispiel Nr. 2).
0,7 (s)
0,9 (s)
1,2—2,3 (m)
2,6 (t)
3,3—3,6 (m)
4,2—4,6 (m)
7,2 (m) 7,8 und 8,3 (s)

Beispiel 3

2,2-Dimethyl-3-benzyloxy-4-(1,2,4-triazol-1-yl)-7-phenylheptan

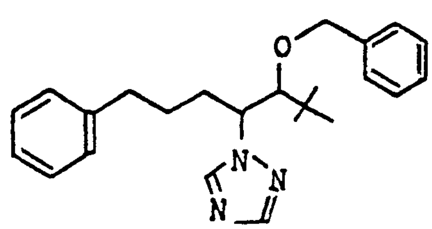

Zu einer Mischung von 40 g (0,14 Mol) 2,2-Dimethyl-4-(1,2,4-triazol-(1)-yl-7-phenylheptanol-(3) und 22,8 g (0,18 Mol) Benzylchlorid in 300 ml Ether und 110 ml DMSO wird 10,8 g Natriumhydrid (80%) in kleinen Portionen zugegeben.

Nach Beendigung der $H_2$-Entwicklung wird zur Vervollständigung der Reaktion auf Rückflußtemperatur erhitzt und 1 Stunde nachgerührt.

Nach Beendigung der Reaktion (DC-Kontrolle) wird das Lösungsmittel abdestilliert, das Reaktionsgemisch abgekühlt und mit Wasser und Ether extrahiert.

Die organische Phase liefert nach Trocknen über Natriumsulfat ein Rohprodukt das durch Destillation bei 216 bis 222°C (0,2 torr) 3,2 g 2,2-Dimethyl-3-Benzyloxy-4-(1,2,4-Triazol-1-yl)-7-phenylheptan liefert. (Wirkstoff Beispiel Nr. 5)

IR-Spektrum
2956, 2868, 1498, 1453, 1274, 1136, 1098, 747, 699, 680 $cm^{-1}$.

Beispiel 4

2,2-Dimethyl-4-(1,2,4-triazol-1-yl)-7-phenylheptanol-(3)
Diastereomeres A (Wirkstoffbeispiel 2a)

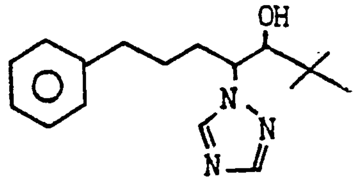

Das gemäß Beispiel 2 synthetisierte 2,2-Dimethyl-4-(1,2,4-triazol-1-yl)-7-phenyl-heptanol-(3) wird an Silikagel mittels Essigester/Cyclohexan chromatographiert. Neben geringem Mengen an Nebenprodukten läßt sich der diastereomere Alkohol 2a spektroskopisch rein abtrennen.

$^1H$—NMR ($CDCl_3$)
7,93 n 8,23 (2H, s)
7,2 (5H, m)
4,5 (1H, m)
3,7—4,2 (breites Signal; —OH)
3,45 (1H, m)
2,6 (2H, t)
0,7 (9H, s)

Beispiel 5

2,2-Dimethyl-4-(1,2,4-triazol-1-yl)-7-phenylheptonal-(3)
Diastereomeres B (Wirkstoffbeispiel 2b)

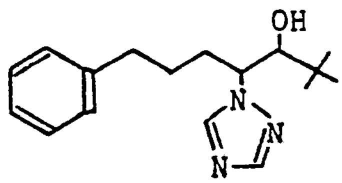

In einer Grignardlösung (bereitet aus 3,15 g Mg-Spänen und 12,45 g 1-Brom-propan in 150 ml Ether) werden bei Raumtemperatur 25 g 2,2-Dimethyl-4-(1,2,4-triazol-1-yl)-7-phenyl-heptenon-(3) in 100 ml Ether getropft. Nach 5 stündigem Rührem bei Rückflußtemperatur wird das Reaktionsgemisch mit 150 ml Ammonchloridlösung versetzt. Die organische Schicht wird abgetrennt und über Natriumsulfat getrocknet.

Beim Einengen kristallisiert 10,5 g 2,2-Dimethyl-4-(1,2,4-triazol-1-yl)-7-phenylheptanol-(3) als diastereomerer Alkohol 2b vom Schmelzpunkt 82—87°C aus.

$^1H$—NMR ($CDCl_3$)
8,05 n.7,95 (2H, s)
7,15 (5H, m)
4,4 (1H, m)
3,53 (1H, m)
3,23 (1H, m; OH)
0,9 (9H, s)

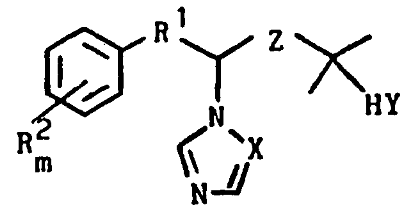

Wirkstoffliste:

| Nr. | $R^1$ | $R^2_m$ | Z | X | HY | Kp °C, mbar | Fp | Diastereomeres |
|---|---|---|---|---|---|---|---|---|
| 1 | $(CH_2)_3$ | H | C=O | N | - | 150 - 154°/0,4 | | |
| 2 | $(CH_2)_3$ | H | CHOH | N | - | 162 - 175°/0,2 | | |
| 2a | $(CH_2)_3$ | H | CHOH | N | - | | | A |
| 2b | $(CH_{23}$ | H | CHOH | N | - | | | B |
| 3 | $(CH_2)_3$ | H | $CHOCH_3$ | N | HCl | 154 - 155°/0,2 | | |
| 4 | $(CH_2)_3$ | H | $CHOCH_2CH=CH$ | N | - | 162 - 170°/0,5 | | |
| 5 | $(CH_2)_3$ | H | $CHOCH_2C_6H_5$ | N | - | 216 - 222°/0,2 | | |
| 6 | $(CH_2)_3$ | H | $CCH_3OH$ | N | - | | 134° | |
| 7 | $(CH_2)_3$ | H | C=O | CH | - | 185 - 187°/0,6 | | |
| 8 | $(CH_2)_3$ | H | CHOH | CH | - | | | |
| 9 | $(CH_2)_3$ | H | $CHOCH_3$ | CH | - | | | |
| 10 | $CH_2CHCH_3CH_2$ | H | C=O | N | - | 160°/0,2 | | |
| 11 | $CH_2CHCH_3CH_2$ | H | CHOH | N | | | | |
| 12 | $(CH_2)_4$ | H | C=O | N | | 186°/0,01 | | |

Wirkstoffliste:

| Nr. | $R^1$ | $R^2_m$ | Z | X | HY | Kp °C, mbar | Fp | Diastereomeres |
|---|---|---|---|---|---|---|---|---|
| 13 | $(CH_2)_4$ | H | CHOH | N | | | | |
| 13a | $(CH_2)_4$ | H | CHOH | N | - | | 96 - 97°C | A |
| 13b | $(CH_2)_4$ | H | CHOH | N | - | | 90 - 91°C | B |
| 14 | $(CH_2)_5$ | H | C=O | N | | 195°/0,05 | | |
| 15 | $(CH_2)_2CHCH_3(CH_2)_2$ | H | C=O | N | | 200°/0,05 | | |
| 16 | $(CH_2)_5$ | H | CHOH | N | | | | |
| 16a | $(CH_2)_5$ | H | CHOH | N | - | | | A |
| 16b | $(CH_2)_5$ | H | CHOH | N | - | | Öl | B |
| 17 | $(CH_2)_5$ | H | C=O | CH | | | | |
| 18 | $(CH_2)_5$ | H | CHOH | CH | | | | |
| 19 | $(CH_2)_3CHCH_3CH_2$ | H | C=O | N | | 179 - 185/0,05 | | |
| 20 | $(CH_2)_3CHCH_3CH_2$ | H | CHOH | N | | | | |
| 20a | $(CH_2)_3CHCH_3-CH_2$ | H | CHOH | N | - | | Öl | A |
| 20b | $(CH_2)_3CHCH_3-CH_2$ | H | CHOH | N | - | | 103 - 104 | B |
| 21 | $(CH_2)_6$ | H | C=O | N | | 180/0,05 | | |
| 22 | $(CH_2)_6$ | H | CHOH | N | | | | |
| 22a | $(CH_2)_6$ | H | CHOH | N | - | | 109 - 110 | A |
| 22b | $(CH_2)_6$ | H | CHOH | N | - | | 100 - 102 | B |
| 23 | $(H_2)_7$ | H | C=O | N | | 210/0,01 | | |
| 24 | $(CH_2)_7$ | H | CHOH | N | | | | |
| 24a | $(CH_2)_7$ | H | CHOH | N | - | | Öl | A |
| 24b | $(CH_2)_7$ | H | CHOH | N | - | | 91 - 94 | B |
| 25 | $(CH_2)_3$ | 4-Cl | C=O | N | | | | |

Wirkstoffliste:

| Nr. | $R^1$ | $R^2_m$ | Z | X | HY | Kp °C, mbar | Fp | Diastereomeres |
|---|---|---|---|---|---|---|---|---|
| 26 | $(CH_2)_3$ | 4-Cl | CHOH | N | | | | |
| 27 | $(CH_2)_4$ | 4-Cl | C=O | N | | | | |
| 28 | $(CH_2)_4$ | 4-Cl | CHOH | N | | | | |
| 29 | $(CH_2)_5$ | 4-Cl | C=O | N | | 205-213/0,1 | | |
| 30 | $(CH_2)_5$ | 4-Cl | CHOH | N | | | | |
| 30a | $(CH_2)_5$ | 4-Cl | CHOH | N | | | Öl | A |
| 30b | $(CH_2)_5$ | 4-Cl | CHOH | N | - | | 99-100 | B |
| 31 | $(CH_2)_3$ | $4-CH_3$ | C=O | N | | | | |
| 32 | $(CH_2)_3$ | $4-CH_3$ | CHOH | N | | | | |
| 33 | $(CH_2)_3$ | 4-Br | C=O | N | | | | |
| 34 | $(CH_2)_3$ | 4-Br | CHOH | N | | | | |
| 35 | $(CH_2)_3$ | 4-F | C=O | N | | | | |
| 36 | $(CH_2)_3$ | 4-F | CHOH | N | | | | |
| 37 | $CH_2CHCH_3CH_2$ | 4-tBu | C=O | N | | 194/0,001 | | |
| 38 | $CH_2CHCH_3CH_2$ | 4-tBu | CHOH | N | | | | |
| 38a | $CH_2CHCH_3CH_2$ | 4-tBu | CHOH | N | - | | Paste | A |
| 38b | $CH_2CHCH_3CH_2$ | 4-tBu | CHOH | N | - | | 112 - 115 | B |
| 39 | $CH_2CHCH_3CH_2$ | $2,4-Cl_2$ | C=O | N | | 118/0,01 | | |
| 40 | $CH_2CHCH_3CH_2$ | $2,4-Cl_2$ | CHOH | N | | | | |
| 40a | $CH_2CHCH_3CH_2$ | $2,4-Cl_2$ | CHOH | N | - | | Öl | A |
| 40b | $CH_2CHCH_3CH_2$ | $2,4-Cl_2$ | CHOH | N | - | | 91 - 93 | B |
| 41 | $(CH_2)_3$ | H | C=O | N | $HNO_3$ | | | |
| 42 | $(CH_2)_3$ | H | $CHOCH_2C_6H_5$ | N | $HNO_3$ | | | |

Wirkstoffliste:

| Nr. | $R^1$ | $R^2_m$ | Z | X | HY | Kp °C, mbar | Fp | Diastereomeres |
|---|---|---|---|---|---|---|---|---|
| 43 | $CH_2$-$CHCH_3CH_2$ | 4-Me | C=O | N | | 156/0,01 | | |
| 44a | $CH_2$-$CHCH_3CH_2$ | 4-Me | CHOH | N | - | | Öl | A |
| 44b | $CH_2$-$CHCH_3CH_2$ | 4-Me | CHOH | N | | | 110 - 114 | B |
| 45 | $CH_2$-$CHCH_3CH_2$ | 4-F | C=O | N | - | 151/0,01 | | |
| 46a | $CH_2$-$CHCH_3CH_2$ | 4-F | CHOH | N | - | | Öl | A |
| 46b | $CH_2$-$CHCH_3CH_2$ | 4-F | CHOH | N | - | | 86 - 88 | B |
| 47 | $CH_2$-$CHCH_3CH_2$ | 4-$OCH_3$ | C=O | N | - | 176/0,005 | | |
| 48a | $CH_2$-$CHCH_3CH_2$ | 4-$OCH_3$ | CHOH | N | - | | Öl | A |
| 48b | $CH_2$-$CHCH_3CH_2$ | 4-$OCH_3$ | CHOH | N | - | | 68 - 70 | B |
| 49 | $(CH_2)_5$ | 4-Me | C=O | N | - | 173/0,01 | | |
| 50a | $(CH_2)_5$ | 4-Me | CHOH | N | - | | Öl | A |
| 50b | $(CH_2)_5$ | 4-Me | CHOH | N | - | | 99 102 | B |
| 51 | $(CH_2)_5$ | 2-Me | C=O | N | - | | | |
| 52a | $(CH_2)_5$ | 2-Me | CHOH | N | - | | | A |
| 52b | $(CH_2)_5$ | 2-Me | CHOH | N | - | | | B |
| 53 | $CH_2CHCH_3CH_2$ | 2-F | C=O | N | - | | | |
| 54a | $CH_2CHCH_3CH_2$ | 2-F | CHOH | N | - | | | A |
| 54b | $CH_2CHCH_3CH_2$ | 2-F | CHOH | N | - | | | B |
| 55a | $(CH_2)CHCH_3(CH_2)$ | H | CHOH | N | - | | Öl | A |
| 55b | $(CH_2)CHCH_3(CH_2)$ | H | CHOH | N | - | | Öl | B |
| 56 | $(CH_2)CHCH_3(CH_2)$ | 4-Cl | C=O | N | - | | | |
| 57a | $CH_2$-$CHCH_3$-$CH_2$ | 4-Cl | CHOH | N | - | | | A |
| 57b | $CH_2$-$CHCH_3$-$CH_2$ | 4-ClH | CHOH | N | - | | | B |

NMR-Werte der diastereomeren Alkohole A und B

| Nr. | Triazol | | Aromat | CH-Thiazol | CH-OH | tert.-Butyl |
|---|---|---|---|---|---|---|
| 2a | 8,23 (1H, s) | 7,93 (1H, s) | 7,2 (5H, m) | 4,5 (1H, m) | 3,45 (1H, m) | 0,7 (9H, s) |
| 2b | 8,03 (1H, s) | 7,95 (1H, s) | 7,15 (5H, m) | 4,4 (1H, m) | 3,53 (1H, m) | 0,9 (9H, s) |
| 16a | 8,10 (1H, s) | 7,93 (1H, s) | 7,15 (5H, m) | 4,4 (1H, m) | 3,4 (1H, m) | 0,65 (9H, s) |
| 16b | 8,1 (1H, s) | 7,9 (1H, s) | 7,2 (5H, m) | 4,38(1H, m) | 3,5 (1H, m) | 0,95 (9H, s) |
| 20a | 8,1 (1H, s) | 7,95 (1H, s) | 7,2 (5H, m) | 4,4 (1H, m) | 3,55 (1H, m) | 0,95 (9H, s) |
| 24b | 8,1 (1H, s) | 7,95 (1H, s) | 7,2 (5H, m) | 4,4 (1H, m) | 3,55 (1H, m) | 0,95 (9H, s) |
| 30a | 8,11 (1H, s) | 7,95 (1H, s) | 7,15(4H,AA'BB') | 4,45(1H, m) | 3,42 (1H, m) | 0,7 (9H, s) |
| 38a | 8,05 (1H, s) | 7,90 (1H, s) | 7,1(4H,AA'BB') | 4,55(1H, m) | 3,4 (1H, m) | 1,3 (9H, s);<br>0,7 (9H, s) |
| 38b | | | | | | 1,3 (9H, s) |
| 40a | 8,15<br>(1H, s)<br>8,10 | 7,95<br>(1H, 1)<br>7,90 | 6,9-7,4(3H,m) | 4,6 (1H, m) | | 0,7<br>(9H)<br>0,65 |
| 44a | | | | | | 0,6 (9H)<br>0,68 |
| 46a | 8,1 (1H, s) | 7,9 (1H, s) | 6,58<br>(höchstes Peak 4H) | 4,6 (1H, m) | | 0,7 (9H, s) |
| 48a | | | | | | 0,6 (9H)<br>0,68 |
| 50a | 8,15 (1H, s) | 7,9 (1H, s) | 7,0(4H,AA'BB') | 4,45(1H, m) | 3,4 (1H) | 0,7 (9H, s) |
| 55a | 8,15 (1H, d) | 7,95 (1H, s) | 7,2 (5H, m) | 4,4 (1H, m) | 3,45 (1H, d) | 0,7 (9H, s) |
| 55b | 8,15 (1H, d) | 7,95 (1H, s) | | | | 0,95 (9H, s) |

[s - Singulett, m - Triplett]

Die Wirksamkeiten der neuen Verbindungen werden aus folgenden Beispielen ersichtlich:

Beispiel I

Wirksamkeit gegen Gurkenmehltau

Blätter von in Töpfen gewachsenen Gurkenkeimlingen der Sorte "Chinesische Schlange" werden im Zweiblattstadium mit einer Sporensuspension des Gurkenmehltaus (Erysiphe cichoracearum) besprüht. Nach etwa 20 Stunden werden die Versuchspflanzen mit wäßriger Spritzbrühe, die 80% Wirkstoff und 20% Emulgiermittel in der Trockensubstanz enthält, bis zur Tropfnässe besprüht. Nach dem Antrocknen des Spritzbelages werden sie anschließend im Gewächshaus bei Temperaturen zwischen 20 und 22°C und 70 bis 80% relativer Luftfeuchtigkeit aufgestellt. Zur Beurteilung der Wirksamkeit der neuen Stoffe wird das Ausmaß der Pilzentwicklung nach 21 Tagen beurteilt.

Bewertung:
0 = kein Pilzbefall, abgestuft bis
5 = Totalbefall

Pflanzenverträglichkeit:
A = leichter Blattschaden

| Wirkstoff Nr. | Befall der Blätter nach Applikation von 0,025 %iger Wirkstoffbrühe |
|---|---|
| 1 | 1 |
| 2 | 0 |
| 3 | 0 |
| 4 | 0 |
| 5 | 0 |
| 6 | 1 |
| 7 | 0 |
| 10 | 0A |
| Vergleichsmittel | 3—4 |
| Unbehandelt | 5 |

Vergleichsmittel: 2,2-Dimethyl-4-(1,2,4-triazol-1-yl)-5-phenylpentanon-3, bekannt aus DE—OS 26 38 470.

Beispiel II

Wirksamkeit gegen Weizenbraunrost

Blätter von in Töpfen gewachsenen Weizensämlingen der Sorte "Jubilar" werden mit Sporen des Braunrostes (Puccinia recondita) bestäubt. Danach werden die Töpfe für 24 Stunden bei 20 bis 22°C in eine Kammer mit hoher Luftfeuchtigkeit (90 bis 95%) gestellt. Während dieser Zeit keimen die Sporen aus und die Keimschläuche dringen in das Blattgewebe ein. Die infizierten Pflanzen werden anschließend mit wäßrigen Spritzbrühen, die 80% Wirkstoff und 20% Emulgiermittel in der Trockensubstanz enthalten, tropfnaß gespritzt. Nach dem Antrocknen des Spritzbelages werden die Versuchspflanzen im

Gewächshaus bei Temperaturen zwischen 20 und 22°C und 65 bis 70% relativer Luftfeuchte aufgestellt. Nach 8 Tagen wird das Ausmaß der Rostpilzentwicklung auf den Blättern ermittelt.

Bewertung:
0 = kein Pilzbefall, abgestuft bis
5 = Totalbefall

| Wirkstoff-Nr. | Befall der Blätter nach Applikation von ... %iger Wirkstoffbrühe | | |
|---|---|---|---|
| 2 | 0 | 1 | 4 |
| 3 | 0 | 0 | 2 |
| 4 | 1 | 1 | 3 |
| 7 | 0 | / | / |
| Vergleichsmittel | 2 | 3 | 3—4 |
| Unbehandelt | 4—5 | | |

Beispiel III

Wirksamkeit gegen Botrytis cinerea an Paprika

Paprikasämlinge der Sorte "Neusiedler Ideal Elite" werden, nachdem sich 4 bis 5 Blätter gut entwickelt haben, mit wäßrigen Suspensionen, die 80% Wirkstoff und 20% Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Bewertung:
0 = kein Pilzbefall, abgestuft bis
5 = Totalbefall

| Wirkstoff-Nr. | Befall der Blätter nach Applikation von 0,05 %iger Wirkstoffbrühe |
|---|---|
| 1 | 1 |
| 2 | 1 |
| 4 | 1 |
| 7 | 1 |
| Vergleichsmittel | 3—4 |
| Unbehandelt | 5 |

Die neuen Verbindungen und ihre Salze und Metallkomplexverbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, Weinbau sowie Gemüse — wie Gurken, Bohnen und Kürbisgewächse-.

Die neuen Verbindungen sind insbesondere geeignet zur Bekämpfung folgender Pflanzenkrankheiten:
Erysiphe graminis (echter Mehltau) in Getreide,
Erysiphe cichoracearum an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,

Venturia inaequalis (Schorf) an Äpfeln, Septoria nodorum an Weizen,
Botrytis cinerea (Grauschimmel) an Erdbeeren, Reben,
Cercospora musae an Bananen,
Pseudocercosporella herpotrichoides an Weizen, Gerste,
Pyricularia oryzae an Reis,
Hemileia vastatrix an Kaffee,
Alternaria solani an Kartoffeln, Tomaten.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle, (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz u.a. zur Bekämpfung holzzerstörender Pilze wie Coniophora puteanea und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung Nr. 1 mit 10 Gewichtsteilen N-Methyl-alphapyrrolidon und erhält eine Lösung, die zur Anwendung in Form kleinster Tropfen geeignet ist.

II. 20 Gewichtsteile der Verbindung Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung Nr. 5 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gewichtsteile der Verbindung Nr. 6 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gewichtsteile der Verbindung Nr. 8 werden den mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

VI. 3 Gewichtsteile der Verbindung Nr. 9 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile der Verbindung Nr. 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit Haftfähigkeit.

VIII. 40 Gewichtsteile der Verbindung Nr. 5 werden mit 10 Teilen Natriumsalz eines Phenolsulfonsäure-harnstoff-formaldehyd-Kondensates, 2 Teilen Kieselgel und 48 Teilen Wasser innig vermisch. Man erhält eine stabile wäßrige Dispersion. Durch Verdünnen mit Wasser erhält man eine wäßrige Dispersion.

IX. 20 Teile der Verbindung Nr. 6 werden mit 2 Teilen Calciumsalz der Dodecylbenzolsulfonsäure, 8

Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden oder auch mit Düngemitteln vermischt und ausgebracht werden. Beim Vermischen mit Fungiziden erhält man dabei in vielen Fällen eine Vergrößerung des fungiziden Wirkungsspektrums.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise: Schwefel, Dithiocarbamate und deren Derivate, wie

Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiouramidsulfide
Ammoniak-Komplex von Zink-(N,N$^a$-ethylen-bis-dithiocarbamat)
Ammoniak-Komplex von Zink-(N,N$^a$-propylen-bis-dithiocarbamat)
Zink-(N,N$^a$-propylen-bis-dithiocarbamat)
N,N$^a$-Polypropylen-bis-(thiocarbamoyl)-disulfid
Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,
heterocyclische Strukturen, wie
2-Heptadecyl-2-imidazolin-acetat
2,4-Dichlor-6-(o-chloranilino)-s-triazin
O,O-Diethyl-phthalimidophosphonothioat
5-Amino--1-(bis-(dimethylamino)-phosphinyl)-3-phenyl-1,2,4-triazol
2,3-Dicyano-1,4-dithiaanthrachinon
2-Thio-1,3-dithio-(4,5,6)-chinoxalin
1-(Butylcarbamoyl)-2-benzimidazol-carbaminsäuremethylester
2-Methoxycarbonylamino-benzimidazol
2-(Furyl-(2)-benzimidazol
2-(Thiazolyl-(4)-benzimidazol
N-(1,1,2,2-Tetrachlorethylthio)-tetrahydrophthalimid
N-Trichlormethylthio-tetrahydrophthalimid
N-Trichlormethyl-phthalimid
N-Dichlorfluormethylthio-N$^a$,N$^a$-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Iod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze

2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1[2-(2,4-Dichlorphenyl)-4-n-propyl-1,3-dioxolan-2-yl-ethyl]-1-H-1,2,4-triazol
N-(n-Propyl)-N-(2,4,6-trichlorphenoxyethyl)-$N^a$-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
α-(2-Chlorphenyl)-α-(4-chlorphenyl)-5-pyrimidin-methanol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol
sowie verschiedene Fungizide, wie
Dodecylguanidinacetat
3-(3-(3,5-Dimethyl-2-oxycyclohexyl)-2-hydroxyethyl)-gluataramid Hexachlorbenzol
DL-Methyl-N-(2,6-dimethyl-phenyl)-N-furoyl(2)-alaninat,
DL-N-(2,6-Dimethyl-phenyl)-N-($2^a$-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-methyl-5-vinyl-3-(3,5-dichlorphenyl)-2,4-dioxo-1,3-oxazolidin
3-(3,5-Dichlorphenyl(-5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureamid.

**Patentansprüche für die Vertragsstaaten: BE CH DE FR GB IT LI NL SE**

1. Azolverbindungen der Formel I

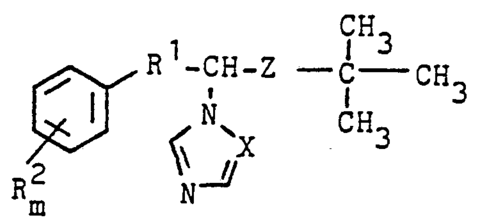

I,

in der bedeuten

$R^1$ eine Alkylenkette mit 3 bis 10 Kohlenstoffatomen, die durch 1 bis 3 $C_1$-bis $C_3$-Alkylreste substituiert sein kann

$R^2$ Wasserstoff, Halogen, $C_1$-bis $C_4$-Alkyl, $C_1$- bis $C_4$-Halogenalkyl, $C_1$- bis $C_4$-Alkoxy, $C_2$- bis $C_5$-Alkenoyl, $C_1$- bis $C_4$-Alkylthio, $C_1$- bis $C_4$-Alkylsulfinyl, $C_1$- bis $C_4$-Alkylsulfonyl, Trifluormethyl, Cyano, Phenoxy oder Benzyloxy

m 1, 2, 3

X CH, N

Z Carbonyl oder die Gruppe

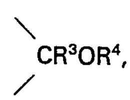

in der $R^3$ für Wasserstoff, $C_1$- bis $C_4$-Alkyl oder $C_2$- bis $C_4$-Alkenyl und $R^4$ für Wasserstoff, $C_1$- bis $C_4$-Alkyl, $C_2$- bis $C_4$-Alkenyl, $C_2$- bis $C_4$-Alkinyl, $C_1$- bis $C_4$-Alkenoyl oder Benzyl steht,
sowie deren für Pflanzen verträgliche Säureadditionssalze und Metallkomplexe.

2. Verfahren zur Herstellung der Azolverbindungen der allgemeinen Formel I gemäß Ansprüch 1, dadurch gekennzeichnet, daß man

a) Ketone der Formel II

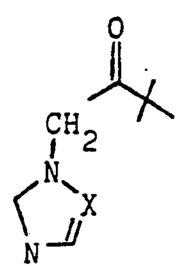

II,

worin X die in Anspruch 1 angeführte Bedeutung hat, oder deren Alkalienolate mit ω-Arylalkylhalogeniden der allgemeinen Formel III

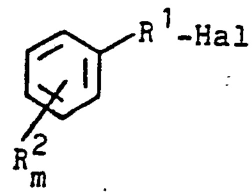

III,

in der $R^1$ und $R^2$ und m die in Anspruch 1 angegebenen Bedeutungen haben und in der Hal Chlor, Brom oder Iod bedeutet, gegebenenfalls in Gegenwart eines Lösungs- und/oder Verdünnungsmittels und/oder anorganischer oder organischer Basen bei Temperaturen zwischen 0 und 200°C umsetzt oder daß man

b) nach an sich bekannten Methoden eine Verbindung der allgemeinen Formel IV

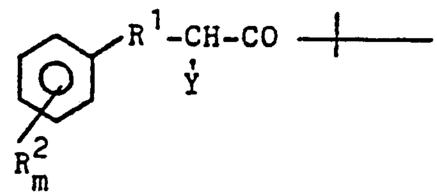

IV,

in der $R^1$, $R^2$ und m die in Anspruch 1 genannten Bedeutungen haben und Y Chlor oder Brom bedeutet, mit Imidazol oder 1,2,4-Triazol gegebenenfalls in Gegenwart eines Lösungs- und/oder Verdünnungsmittels und/oder anorganischer oder organischer Basen bei Temperaturen zwischen 0 und 100°C umsetzt und gewünschtenfalls

c) die so erhaltene Verbindung der Formel I, in der Z für eine CO-Gruppe steht, durch Einwirkung eines komplexen Hydrids oder einer Alkyl-Grignardverbindung mit Wasserstoff in beta-Stellung des Alkylrestes oder durch Einwirkung von Wasserstoff in Gegenwart eines Hydrierungskatalysators, gegebenenfalls in Gegenwart eines Lösungs- oder Verdünnungsmittels, bei Temperaturen zwischen 0 und 100°C zum sekundären Alkohol der Formel I, in der Z für eine CHOH-Gruppe steht, reduziert oder

d) mit einer Grignardverbindung der Formel VII

$$R^3\text{-MgHal} \qquad \text{VII}$$

in der $R^3$ für $C_1$- bis $C_4$-Alkyl oder $C_2$- bis $C_4$-Alkenyl steht und in der Hal Chlor, Brom oder Ion bedeutet, gegebenenfalls in Gegenwart eines Lösungs- oder Verdünnungsmittels und gegebenenfalls in Gegenwart eines Magnesium- oder Tetraalkylammoniumhalogenids bei Temperaturen zwischen 0 und 100°C umsetzt und die so entstandenen Alkoholate zu den tertiären Alkoholen hydrolysiert und die so nach c) erhaltenen sekundären oder nach d) erhaltenen tertiären Alkohole — falls gewünscht — mit einem $C_1$- bis $C_4$-Alkanoylchlorid oder einem $C_1$- bis $C_4$-Alkanoylanhydrid gegebenenfalls in Gegenwart eines Lösungs- oder Verdünnungsmittels und/oder einer anorganischen oder organischen Base und/oder eines Acylierungskatalysators bei Temperaturen zwischen 0 und 100°C oder diese oder deren Alkali- oder deren quartären Ammoniumsalze mit einem Alkylierungsmittel der Formel VIII

$$L\text{—}R^4 \qquad \text{VIII}$$

in der $R^4$ $C_1$- bis $C_4$-Alkyl, $C_2$- bis $C_4$-Alkenyl oder $C_2$- bis $C_4$-Alkinyl bedeutet und L für eine nucleophil verdrängbare Abgangsgruppe steht, gegebenenfalls in Gegenwart eines Lösungs- oder Verdünnungsmittels und/oder anorganischer oder organischer Basen und/oder eines Reaktionsbeschleunigers bei Temperaturen zwischen 0 und 100°C umsetzt oder daß man

e) ein Grignardreagenz der allgemeinen Formel

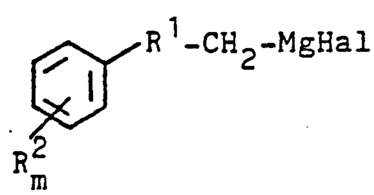

V,

in der $R^1$, $R^2$ und m die in Anspruch 1 genannten Bedeutungen haben und Hal für Chlor, Brom oder Iod steht, mit tert.-Butyl-cyanid nach an sich bekannten Methoden zu dem entsprechenden Keton VI

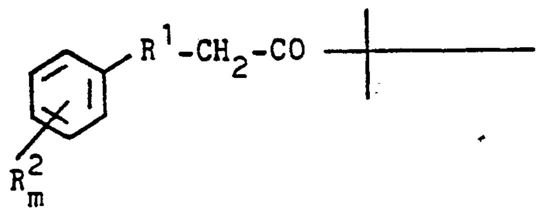

VI

umsetzt, dieses zu Verbindungen der Formel IV halogeniert und das so erhaltene Halogenid mit Imidazol oder 1,2,4-Triazol gegebenenfalls in Gegenwart eines Lösungs- oder Verdünnungsmittel und/oder anorganischer oder organischer Basen bei Temperaturen zwischen 0 und 100°C umsetzt und gewünschtenfalls weiter nach c) oder d) verfährt, worauf man gewünschtenfalls die jeweils erhaltenen Verbindungen der Formel I in ihre für Pflanzen verträgliche Säureadditionssalze oder Metallkomplexe überführt.

3. Fungizide Mittel, enthaltend eine Verbindung gemäß Anspruch 1.

4. Fungizide Mittel, enthaltend eine Verbindung gemäß Anspruch 1 und einen festen oder flüssigen Trägerstoff.

5. Verfahren zur Herstellung von Fungiziden, dadurch gekennzeichnet, daß man mindestens eine Verbindung der Formel I gemäß Anspruch 1 mit festen oder flüssigen Trägerstoffen mischt.

6. Verfahren zur Bekämpfung von Fungi, dadurch gekennzeichnet, daß man mindestens eine Verbindung der Formel I gemäß Anspruch 1 auf diese einwirken läßt.

7. Verfahren zur vorbeugenden Bekämpfung von Fungi, dadurch gekennzeichnet, daß man mindestens eine Verbindung der Formel I gemäß Anspruch 1 auf durch Pilzbefall bedrohte Flächen, Pflanzen oder Saatgüter einwirken läßt.

**Patentansprüche für den Vertragsstaat: AT**

1. Fungizide Mittel, enthaltend eine Azolverbindung der Formel I

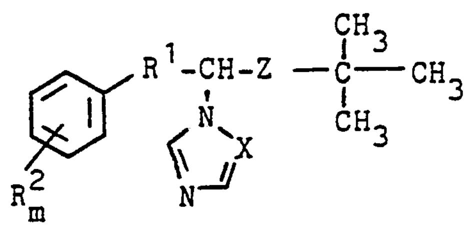

I,

in der bedeuten

$R^1$ eine Alkylenkette mit 3 bis 10 Kohlenstoffatomen, die durch 1 bis 3 $C_1$-bis $C_3$-Alkylreste substituiert sein kann

$R^2$ Wasserstoff, Halogen, $C_1$-bis $C_4$-Alkyl, $C_1$- bis $C_4$-Halogenalkyl, $C_1$- bis $C_4$-Alkoxy, $C_2$- bis $C_5$-Alkenoyl, $C_1$- bis $C_4$-Alkylthio, $C_1$- bis $C_4$-Alkylsulfinyl, $C_1$- bis $C_4$-Alkylsulfonyl, Trifluormethyl, Cyano, Phenoxy oder Benzyloxy

m 1, 2, 3

X CH, N

Z Carbonyl oder die Gruppe

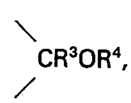

in der $R^3$ für Wasserstoff, $C_1$- bis $C_4$-Alkyl oder $C_2$- bis $C_4$-Alkenyl und $R^4$ für Wasserstoff, $C_1$- bis $C_4$-Alkyl, $C_2$- bis $C_4$-Alkenyl, $C_2$- bis $C_4$-Alkinyl, $C_1$- bis $C_4$-Alkenoyl oder Benzyl steht,
oder deren für Pflanzen verträgliche Säureadditionssalze und Metallkomplexe.

2. Fungizide Mittel, enthaltend eine Verbindung gemäß Anspruch 1 und einen festen oder flüssigen Trägerstoff.

3. Verfahren zur Herstellung von Fungiziden, dadurch gekennzeichnet, daß man mindestens eine Verbindung der Formel I gemäß Anspruch 1 mit festen oder flüssigen Trägerstoffen mischt.

4. Verfahren zur Bekämpfung von Fungi, dadurch gekennzeichnet, daß man mindestens eine Verbindung der Formel I gemäß Anspruch 1 auf diese einwirken läßt.

5. Verfahren zur vorbeugenden Bekämpfung von Fungi, dadurch gekennzeichnet, daß man mindestens

eine Verbindung der Formel I gemäß Anspruch 1 auf durch Pilzbefall bedrohte Flächen, Pflanzen oder Saatgüter einwirken läßt.

6. Verfahren zur Herstellung der Azolverbindungen der allgemeinen Formel I gemäß Anspruch 1, dadurch gekennzeichnet, daß man

a) Ketone der Formel II

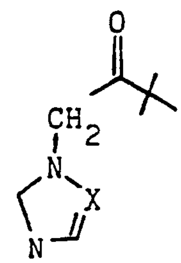

II,

worin X die in Anspruch 1 angeführte Bedeutung hat, oder deren Alkalienolate mit ω-Arylalkylhalogeniden der allgemeinen Formel III

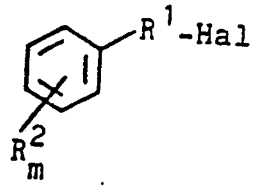

III,

in der $R^1$ und $R^2$ und m die in Anspruch 1 angegebenen Bedeutungen haben und in der Hal Chlor, Brom oder Iod bedeutet, gegebenenfalls in Gegenwart eines Lösungs- und/oder Verdünnungsmittels und/oder anorganischer oder organischer Basen bei Temperaturen zwischen 0 und 200°C umsetzt oder daß man

b) nach an sich bekannten Methoden eine Verbindung der allgemeinen Formel IV

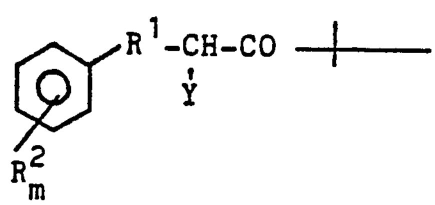

IV,

in der $R^1$, $R^2$ und m die in Anspruch 1 genannten Bedeutungen haben und Y Chlor oder Brom bedeutet, mit Imidazol oder 1,2,4-Triazol gegebenenfalls in Gegenwart eines Lösungs- und/oder Verdünnungsmittels und/oder anorganischer oder organischer Basen bei Temperaturen zwischen 0 und 100°C umsetzt und gewünschtenfalls

c) die so erhaltene Verbindung der Formel I, in der Z für eine CO-Gruppe steht, durch Einwirkung eines komplexen Hydrids oder einer Alkyl-Grignardverbindung mit Wasserstoff in beta-Stellung des Alkylrestes oder durch Einwirkung von Wasserstoff in Gegenwart eines Hydrierungskatalysators, gegebenenfalls in Gegenwart eines Lösungs- oder Verdünnungsmittels, bei Temperaturen zwischen 0 und 100°C zum sekundären Alkohol der Formel I, in der Z für eine CHOH-Gruppe steht, reduziert oder

d) mit einer Grignardverbindung der Formel VII

$R^3$-MgHal VII

in der $R^3$ für $C_1$- bis $C_4$-Alkyl oder $C_2$- bis $C_4$-Alkenyl steht und in der Hal Chlor, Brom oder Ion bedeutet, gegebenenfalls in Gegenwart eines Lösungs- oder Verdünnungsmittels und gegebenenfalls in Gegenwart eines Magnesium- oder Tetraalkylammoniumhalogenids bei Temperaturen zwischen 0 und 100°C umsetzt und die so entstandenen Alkoholate zu den tertiären Alkoholen hydrolysiert und die so nach c) erhaltenen sekundären oder nach d) erhaltenen tertiären Alkohole — falls gewünscht — mit einem $C_1$- bis $C_4$-Alkanoylchlorid oder einem $C_1$- bis $C_4$-Alkanoylanhydrid gegebenenfalls in Gegenwart eines Lösungs- oder Verdünnungsmittels und/oder einer anorganischen oder organischen Base und/oder eines Acylierungskatalysators bei Temperaturen zwischen 0 und 100°C oder diese oder deren Alkali- oder deren quartären Ammoniumsalze mit einem Alkylierungsmittel der Formel VIII

L—$R^4$ VIII

in der $R^4$ $C_1$- bis $C_4$-Alkyl, $C_2$- bis $C_4$-Alkenyl oder $C_2$- bis $C_4$-Alkinyl bedeutet und L für eine nucleophil verdrängbare Abgangsgruppe steht, gegebenenfalls in Gegenwart eines Lösungs- oder Verdünnungsmittels und/oder anorganischer oder organischer Basen und/oder eines Reaktionsbeschleunigers bei Temperaturen zwischen 0 und 100°C umsetzt oder daß man

e) ein Grignardreagenz der allgemeinen Formel

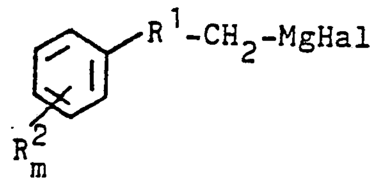

V,

in der $R^1$, $R^2$ und m die in Anspruch 1 genannten Bedeutungen haben und Hal für Chlor, Brom oder Iod steht, mit tert.-Butyl-cyanid nach an sich bekannten Methoden zu dem entsprechenden Keton VI

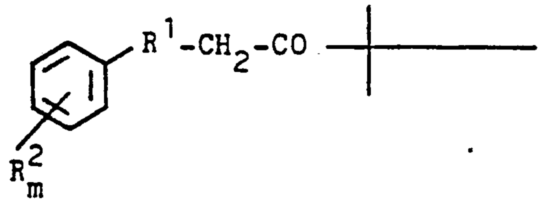

VI

umsetzt, dieses zu Verbindungen der Formel IV halogeniert und das so erhaltene Halogenid mit Imidazol oder 1,2,4-Triazol gegebenenfalls in Gegenwart eines Lösungs- oder Verdünnungsmittel und/oder anorganischer oder organischer Basen bei Temperaturen zwischen 0 und 100°C umsetzt und gewünschtenfalls weiter nach c) oder d) verfährt, worauf man gewünschtenfalls die jeweils erhaltenen Verbindungen der Formel I in ihre für Pflanzen verträgliche Säureadditionssalze oder Metallkomplexe überführt.

**Revendications pour les Etats contractants: BE CH DE FR GB IT LI NL SE**

1. Dérivés azoliques de formule I

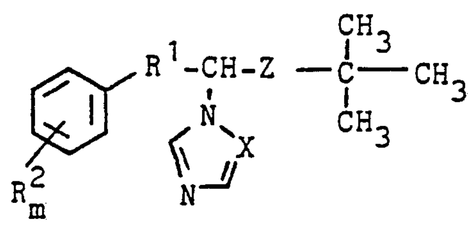

I,

dans laquelle

$R^1$ représente une chaîne alkylène en $C_3$—$C_{10}$ qui peut être substituée par un à trois groupes alkyle en $C_1$—$C_3$,

$R^2$ représente l'hydrogène, un halogène, un groupe alkyle en $C_1$—$C_4$, halogénoalkyle en $C_1$—$C_4$, alcoxy en $C_1$—$C_4$, alcanoyle en $C_2$—$C_5$, alkylthio en $C_1$—$C_4$, alkylsulfinyle en $C_1$—$C_4$, alkylsulfonyle en $C_1$—$C_4$, trifluorométhyle, cyano, phénoxy ou benzyloxy,

m est égal à 1, 2 ou 3

X représente CH, N

Z représente un groupe carbonyle ou le groupe

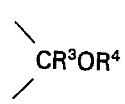

dans laquel $R^3$ représente l'hydrogène, un groupe alkyle en $C_1$—$C_4$ ou alcényle en $C_2$—$C_4$ et $R^4$ représente l'hydrogène, un groupe alkyle en $C_1$—$C_4$, alcényle en $C_2$—$C_4$, alcynyle en $C_2$—$C_4$, alcényle en $C_1$—$C_4$ ou benzyle,

et leur sels formés par addition avec des acides et complexes métalliques tolérés par les végétaux.

2. Procédé de préparation des dérivés azoliques de formule générale I de la revendication 1, caractérisé par le fait que:

a) on fait réagir des cétones de formule II

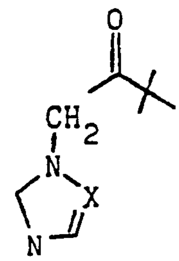

II,

dans laquelle X a les significations indiquées dans la revendication 1, ou leurs énolates alcalins, avec des halogénures d'oméga-arylalkyle de formule générale III

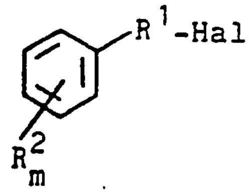

III,

dans laquelle $R^1$, $R^2$ et m ont les significations indiquées dans la revendication 1 et Hal représente le chlore, le brome ou l'iode, éventuellement en présence d'un solvant et/ou diluant et/ou de bases minérales ou organiques, à des températures de 0 à 200°C, ou bien par le fait que

b) on fait réagir par des modes opératoires connus en soi un composé de formule générale IV

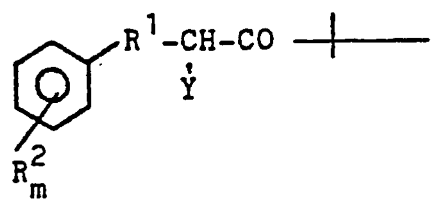

IV,

dans laquelle $R^1$, $R^2$ et m ont les significations indiquées dans la revendication 1 et Y représente le chlore ou le brome, avec l'imidazole ou le 1,2,4-triazole, éventuellement en présence d'un solvant et/ou diluant et/ou de bases minérales ou organiques, à des températures de 0 à 100°C, et si on le désire

c) on réduit un composé ainsi obtenu, répondant à la formule I dans laquelle Z représente un groupe CO, par action d'un hydrure complexe ou d'un dérivé alkylique de Grignard contenant de l'hydrogène en position bêta du groupe alkyle, ou par action de l'hydrogène en présence d'un catalyseur d'hydrogénation, éventuellement en présence d'un solvant ou diluant, à des températures de 0 à 100°C, ce qui donne l'alcool secondaire de formule I dans laquelle Z représente un groupe CHOH, ou bien

d) on le fait réagir avec un dérivé de Grignard de formule VII

$$R^3\text{-MgHal} \qquad VII$$

dans laquelle $R^3$ représente un groupe alkyle en $C_1$—$C_4$ ou alcényle en $C_2$—$C_4$ et Hal représente le chlore, le brome ou l'iode, éventuellement en présence d'un solvant ou diluant et éventuellement en présence d'un halogénure de magnésium ou de tétraalkylammonium, à des températures de 0 à 100°C, ce qui donne un alcoolate qu'on hydrolyse en l'alcool tertiare, après quoi on fait réagir l'alcool secondaire obtenu selon c) ou l'alcool tertiaire obtenu selon d) — si on le désire — avec un chlorure d'alcanoyle en $C_1$—$C_4$ ou un anhydride d'alcanoyle en $C_1$—$C_4$, éventuellement en présence d'un solvant ou diluant et/ou d'une base minérale ou organique et/ou d'un catalyseur d'acylation, à des températures de 0 à 100°C, ou bien on fait réagir ces alcools on leurs sels alcalins ou d'ammonium quaternaire avec un agent alkylant de formule VIII

$$L—R^4 \qquad VIII$$

dans laquelle $R^4$ représente un groupe alkyle en $C_1$—$C_4$, alcényle en $C_2$—$C_4$ ou alcynyle en $C_2$—$C_4$ et L représente un groupe éliminable par un agent nucléophile, éventuellement en présence d'un solvant ou

diluant et/ou de bases minérales ou organiques et/ou d'un accélérateur de réaction, à des températures de 0 à 100°C, ou bien en ce que

e) on convertit un réactif de Grignard de formule générale

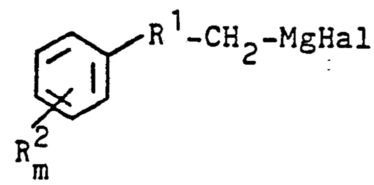

V,

dans laquelle $R^1$, $R^2$ et m ont les significations indiquées dans la revendication 1 et Hal représente le chlore, le brome ou l'iode, par réaction avec le cyanure de tert-butyle, selon des modes opératoires connus en soi, en la cétone correspondante VI

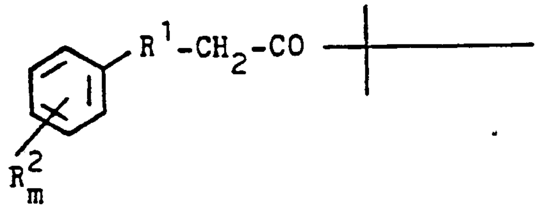

VI

qu'on convertit par halogénation en un composé de formule IV, après quoi on fait réagir cet halogénure avec l'imidazole ou le 1,2,4-triazole, éventuellement en présence d'un solvant ou diluant et/ou de bases minérales ou organiques, à des températures de 0 à 100°C, et si on le désire, on poursuit comme décrit en c) ou d) ci-dessus,

après quoi, si on le désire, on convertit les composés obtenus dans chaque cas, répondant à la formule I, en leurs sels formés par addition avec des acides et complexes métalliques tolérés par les végétaux.

3. Produits fongicides contenant un composé selon la revendication 1.

4. Produits fongicides contenant un composé selon la revendication 1 et un véhicule solide ou liquide.

5. Procédé de préparation de produits fongicides, caractérisé en ce que l'on mélange au moins un composé de formule I selon la revendication 1 avec des véhicules solides ou liquides.

6. Procédé pour combattre les mycètes, caractérisé par le fait que l'on fait agir sur les mycètes au moins un composé de formule I selon la revendication 1.

7. Procédé pour combattre préventivement des mycètes, caractérisé par le fait que l'on fait agir au moins un composé de formule I selon la revendication 1 sur les surfaces, les végétaux ou les semences menacés d'une attaque par les mycètes.

**Revendications pour l'Etat contractant: AT**

1. Produits fongicides, contenant un dérivé azolique de formule I

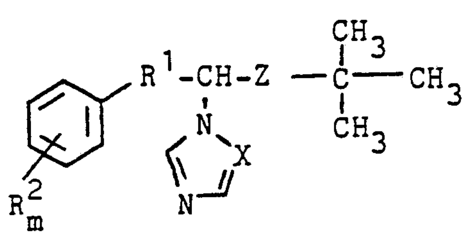

I,

dans laquelle

$R^1$ représente une chaîne alkylène en $C_3$—$C_{10}$ qui peut être substituée par un à trois groupes alkyle en $C_1$—$C_3$,

$R^2$ représente l'hydrogène, un halogène, un groupe alkyle en $C_1$—$C_4$, halogénoalkyle en $C_1$—$C_4$, alcoxy en $C_1$—$C_4$, alcanoyle en $C_2$—$C_5$, alkylthio en $C_1$—$C_4$, alkylsulfinyle en $C_1$—$C_4$, alkylsulfonyle en $C_1$—$C_4$, trifluorométhyle, cyano, phénoxy ou benzyloxy,

m est égal à 1, 2 ou 3

X représente CH, N

Z représente un groupe carbonyle ou le groupe $CR^3OR^4$ dans laquel $R^3$ représente l'hydrogène, un groupe alkyle en $C_1$—$C_4$ ou alcényle en $C_2$—$C_4$ et $R^4$ représente l'hydrogène, un groupe alkyle en $C_1$—$C_4$, alcényle en $C_2$—$C_4$, alcynyle en $C_2$—$C_4$, alcényle en $C_1$—$C_4$ ou benzyle,

ou leur sels formés par addition avec des acides ou complexes métalliques tolérés par les végétaux.

2. Produits fongicides contenant un composé selon la revendication 1 et un véhicule solide ou liquide.

3. Procédé de préparation de produits fongicides, caractérisé en ce que l'on mélange au moins un

composé de formule I selon la revendication 1 avec des véhicules solides ou liquides.

4. Procédé pour combattre les mycètes, caractérisé par le fait que l'on fait agir sur les mycètes au moins un composé de formule I selon la revendication 1.

5. Procédé pour combattre préventivement des mycètes, caractérisé par le fait que l'on fait agir au moins un composé de formule I selon la revendication 1 sur les surfaces, les végétaux ou les semences menacés d'une attaque par les mycètes.

6. Procédé de préparation des dérivés azoliques de formule générale I de la revendication 1, caractérisé par le fait que:

a) on fait réagir des cétones de formule II

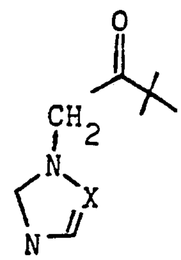

II,

dans laquelle X a les significations indiquées dans la revendication 1, ou leurs énolates alcalins, avec des halogénures d'oméga-arylalkyle de formule générale III

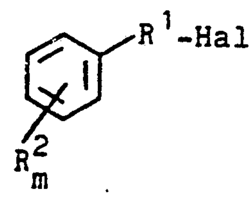

III,

dans laquelle $R^1$, $R^2$ et m ont les significations indiquées dans la revendication 1 et Hal représente le chlore, le brome ou l'iode, éventuellement en présence d'un solvant et/ou diluant et/ou de bases minérales ou organiques, à des températures de 0 à 200°C, ou bien par le fait que

b) on fait réagir par des modes opératoires connus en soi un composé de formule générale IV

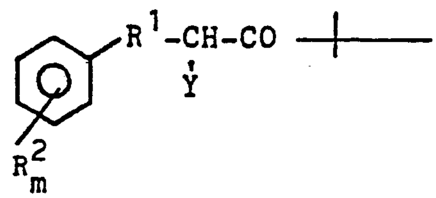

IV,

dans laquelle $R^1$, $R^2$ et m ont les significations indiquées dans la revendication 1 et Y représente le chlore ou le brome, avec l'imidazole ou le 1,2,4-triazole, éventuellement en présence d'un solvant et/ou diluant et/ou de bases minérales ou organiques, à des températures de 0 à 100°C, et si on le désire

c) on réduit un composé ainsi obtenu, répondant à la formule I dans laquelle Z représente un groupe CO, par action d'un hydrure complexe ou d'un dérivé alkylique de Grignard contenant de l'hydrogène en position bêta du groupe alkyle, ou par action de l'hydrogène en présence d'un catalyseur d'hydrogènation, éventuellement en présence d'un solvant ou diluant, à des températures de 0 à 100°C, ce qui donne l'alcool secondaire de formule I dans laquelle Z représente un groupe CHOH, ou bien

d) on le fait réagir avec un dérivé de Grignard de formule VII

$$R^3\text{-MgHal} \qquad \text{VII}$$

dans laquelle $R^3$ représente un groupe alkyle en $C_1$—$C_4$ ou alcényle en $C_2$—$C_4$ et Hal représente le chlore, le brome ou l'iode, éventuellement en présence d'un solvant ou diluant et éventuellement en présence d'un halogénure de magnésium ou de tétraalkylammonium, à des températures de 0 à 100°C, ce qui donne un alcoolate qu'on hydrolyse en l'alcool tertiaire, après quoi on fait réagir l'alcool secondaire obtenu selon c) ou l'alcool tertiaire obtenu selon d) — si on le désire — avec un chlorure d'alcanoyle en $C_1$—$C_4$ ou un anhydride d'alcanoyle en $C_1$—$C_4$, éventuellement en présence d'un solvant ou diluant et/ou d'une base minérale ou organique et/ou d'un catalyseur d'acylation, à des températures de 0 à 100°C, ou bien on fait réagir ces alcools on leurs sels alcalins ou d'ammonium quaternaire avec un agent alkylant de formule VIII

$$L\text{—}R^4 \qquad \text{VIII}$$

dans laquelle $R^4$ représente un groupe alkyle en $C_1$—$C_4$, alcényle en $C_2$—$C_4$ ou alcynyle en $C_2$—$C_4$ et L représente un groupe éliminable par un agent nucléophile, éventuellement en présence d'un solvant ou diluant et/ou de bases minérales ou organiques et/ou d'un accélérateur de réaction, à des températures de 0 à 100°C, ou bien en ce que

e) on convertit un réactif de Grignard de formule générale

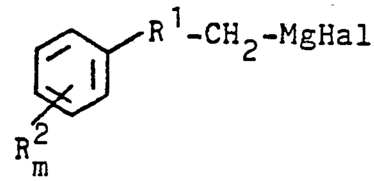

V,

dans laquelle $R^1$, $R^2$ et m ont les significations indiquées dans la revendication 1 et Hal représente le chlore, le brome ou l'iode, par réaction avec le cyanure de tert-butyle, selon des modes opératoires connus en soi, en la cétone correspondante VI

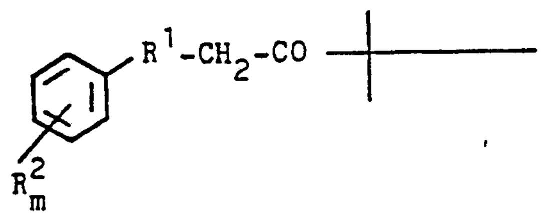

VI

qu'on convertit par halogénation en un composé de formule IV, après quoi on fait réagir cet halogénure avec l'imidazole ou le 1,2,4-triazole, éventuellement en présence d'un solvant ou diluant et/ou de bases minérales ou organiques, à des températures de 0 à 100°C, et si on le désire, on poursuit comme décrit en c) ou d) ci-dessus,

après quoi, si on le désire, on convertit les composés obtenus dans chaque cas, répondant à la formule I, en leurs sels formés par addition avec des acides et complexes métalliques tolérés par les végétaux.

**Claims for the Contracting States: BE CH DE FR GB IT LI NL SE**

1. An azole compound of the formula I

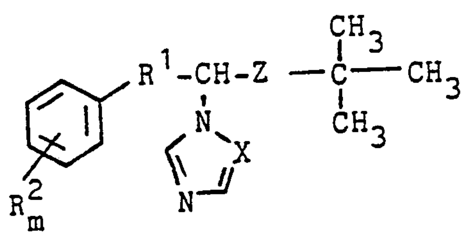

I,

where

$R^1$ is an alkylene chain of 3 to 10 carbon atoms which may be substituted by 1 to 3 $C_1$—$C_3$-alkyl radicals,

$R^2$ is hydrogen, halogen, $C_1$—$C_4$-alkyl, $C_1$—$C_4$-haloalkyl, $C_1$—$C_4$-alkoxy, $C_2$—$C_5$-alkenoyl, $C_1$—$C_4$-alkylthio, $C_1$—$C_4$-alkylsulfinyl, $C_1$—$C_4$-alkylsulfonyl, trifluoromethyl, cyano, phenoxy or benzyloxy,

m is 1, 2 or 3,

X is CH or N, and

Z is carbonyl or the group, $CR^3OR^4$, where $R^3$ is hydrogen, $C_1$—$C_4$-alkyl or $C_2$—$C_4$-alkenyl and $R^4$ is hydrogen, $C_1$—$C_4$-alkyl, $C_2$—$C_4$-alkenyl, $C_2$—$C_4$-alkynyl, $C_1$—$C_4$-alkenoyl or benzyl,

and their plant-tolerated acid addition salts and metal complexes.

2. A process for the preparation of an azole compound of the formula I as claimed in claim 1, wherein

a) a ketone of the formula II

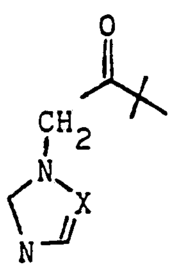

II,

where X has the meanings stated in claim 1, or its alkali metal anolate is reacted with an ω-arylalkyl halide of the formula III

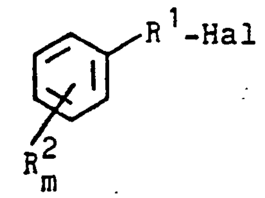

III,

where $R^1$ and $R^2$ and m have the meanings stated in claim 1 and Hal is chlorine, bromine or iodine, in the presence or absence of a solvent and/or of a diluent and/or of inorganic or organic bases at from 0 to 200°C, or

b) a compound of the formula IV

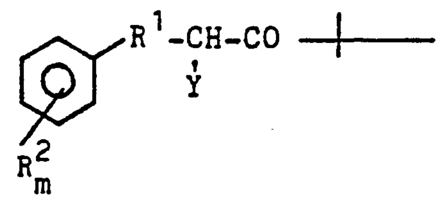

IV,

where $R^1$, $R^2$ and m have the meanings stated in claim 1 and Y is chlorine or bromine, is reacted by a conventional method with imidazole or 1,2,4-triazole in the presence or absence of a solvent and/or of a diluent and/or of inorganic or organic bases at from 0 to 100°C, and, if desired,

c) the resulting compound of the formula I, where Z is CO, is reduced to a secondary alcohol of the formula I where Z is CHOH by the action of a complex hydride or of an alkyl Grignard compound having hydrogen in the beta-position of the alkyl radical or by the action of hydrogen in the presence of a hydrogenation catalyst, in the presence or absence of a solvent or of a diluent, at from 0 to 100°C, or

d) is reacted with a Grignard compound of the formula VII

$$R^3\text{—MgHal} \qquad \text{VII}$$

where $R^3$ is $C_1$—$C_4$-alkyl or $C_2$—$C_4$-alkenyl and Hal is chlorine, bromine or iodine, in the presence or absence of a solvent or of a diluent and in the presence or absence of a magnesium or tetraalkylammonium halide at from 0 to 100°C, and the resulting alcoholate is hydrolyzed to a tertiary alcohol, and the secondary alcohol obtained in c) or the tertiary alcohol obtained in d) is, if desired, reacted with a $C_1$—$C_4$-alkanoyl chloride or with a $C_1$—$C_4$-alkanoyl anhydride in the presence or absence of a solvent or of a diluent and/or of an inorganic or organic base and/or of an acylation catalyst at from 0 to 100°C, or said alcohol or its alkali metal or quaternary ammonium salt is reacted with an alkylating agent of the formula VIII

$$L\text{—}R^4 \qquad \text{VIII}$$

where $R^4$ is $C_1$—$C_4$-alkyl, $C_2$—$C_4$-alkenyl or $C_2$—$C_4$-alkynyl and L is a nucleophilically displaceable leaving group, in the presence or absence of a solvent or of a diluent and/or of inorganic or organic bases and/or of a reaction accelerator at from 0 to 100°C, or

e) a Grignard reagent of the formula

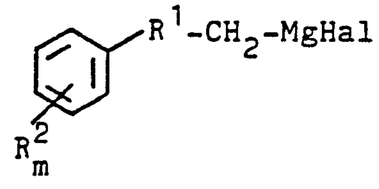

V,

where $R^1$, $R^2$ and m have the meanings stated in claim 1 and Hal is chlorine, bromine or iodine, is reacted with tert-butyl cyanide by a conventional method to give the corresponding ketone VI

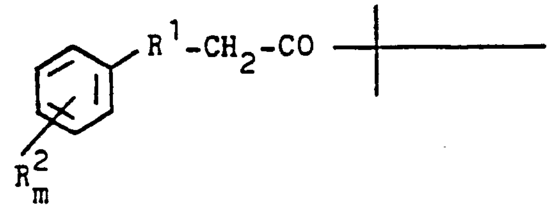

VI

and the latter is halogenated to give a compound of the formula IV and the resulting halide is reacted with imidazole or 1,2,4-triazole in the presence or absence of a solvent or of a diluent and/or of inorganic or organic bases at from 0 to 100°C and, if desired, the procedure is continued according to c) or d), after which, if desired, the resulting compound of the formula I is converted into its plant-tolerated acid addition salts or metal complexes.

3. A fungicide containing a compound as claimed in claim 1.

4. A fungicide containing a compound as claimed in claim 1 and a solid or liquid carrier.

5. A process for the preparation of a fungicide, wherein one or more compounds of the formula I as claimed in claim 1 are mixed with solid or liquid carriers.

6. A method for controlling fungi, wherein one or more compounds of the formula I as claimed in claim 1 are allowed to act on the fungi.

7. A method for the preventive control of fungi, wherein one or more compounds of the formula I as claimed in claim 1 are allowed to act on surfaces, plants or seed threatened by fungal attack.

**Claims for the Contracting State: AT**

1. A fungicide containing an azole compound of the formula I

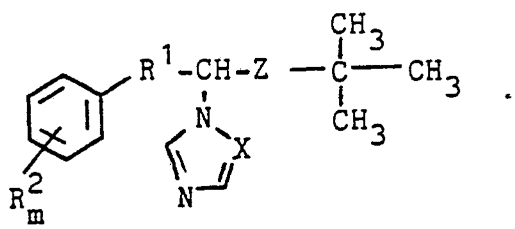

I,

where

$R^1$ is an alkylene chain of 3 to 10 carbon atoms which may be substituted by 1 to 3 $C_1$—$C_3$-alkyl radicals,

$R^2$ is hydrogen, halogen, $C_1$—$C_4$-alkyl, $C_1$—$C_4$-haloalkyl, $C_1$—$C_4$-alkoxy, $C_2$—$C_5$-alkenoyl, $C_1$—$C_4$-alkylthio, $C_1$—$C_4$-alkylsulfinyl, $C_1$—$C_4$-alkylsulfonyl, trifluoromethyl, cyano, phenoxy or benzyloxy,

m is 1, 2 or 3,

X is CH or N, and

Z is carbonyl or the group, $CR^3OR^4$, where $R^3$ is hydrogen, $C_1$—$C_4$-alkyl or $C_2$—$C_4$-alkenyl and $R^4$ is hydrogen, $C_1$—$C_4$-alkyl, $C_2$—$C_4$-alkenyl, $C_2$—$C_4$-alkynyl, $C_1$—$C_4$-alkenoyl or benzyl,

or their plant-tolerated acid addition salts or metal complexes.

2. A fungicide containing a compound as claimed in claim 1 and a solid or liquid carrier.

3. A process for the preparation of a fungicide, wherein one or more compounds of the formula I as claimed in claim 1 are mixed with solid or liquid carriers.

4. A method for controlling fungi, wherein one or more compounds of the formula I as claimed in claim 1 are allowed to act on the fungi.

5. A method for the preventive control of fungi, wherein one or more compounds of the formula I as claimed in claim 1 are allowed to act on surfaces, plants or seed threatened by fungal attack.

6. A process for the preparation of an azole compound of the formula I as claimed in claim 1, wherein

a) a ketone of the formula II

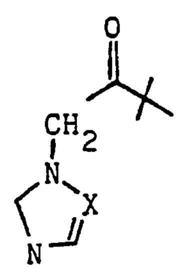

II,

where X has the meanings stated in claim 1, or its alkali metal enolate is reacted with an ω-arylalkyl halide of the formula III

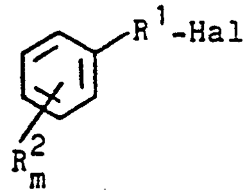

III,

where $R^1$ and $R^2$ and m have the meanings stated in claim 1 and Hal is chlorine, bromine or iodine, in the presence or absence of a solvent and/or of a diluent and/or of inorganic or organic bases at from 0 to 200°C, or

b) a compound of the formula IV

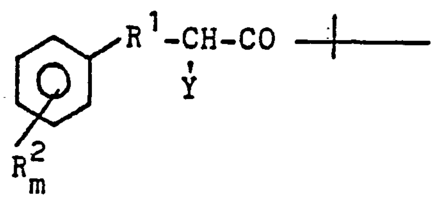

IV,

where $R^1$, $R^2$ and m have the meanings stated in claim 1 and Y is chlorine or bromine, is reacted by a conventional method with imidazole or 1,2,4-triazole in the presence or absence of a solvent and/or of a diluent and/or of inorganic or organic bases at from 0 to 100°C, and, if desired,

c) the resulting compound of the formula I, where Z is CO, is reduced to a secondary alcohol of the formula I where Z is CHOH by the action of a complex hydride or of an alkyl Grignard compound having hydrogen in the beta-position of the alkyl radical or by the reaction of hydrogen in the presence of a hydrogenation catalyst, in the presence or absence of a solvent or of a diluent, at from 0 to 100°C, or

d) is reacted with a Grignard compound of the formula VII

$$R^3\text{—MgHal} \qquad \text{VII}$$

where $R^3$ is $C_1$—$C_4$-alkyl or $C_2$—$C_4$-alkenyl and Hal is chlorine, bromine or iodine, in the presence or absence of a solvent or of a diluent and in the presence or absence of a magnesium or tetraalkylammonium halide at from 0 to 100°C, and the resulting alcoholate is hydrolyzed to a tertiary alcohol, and the secondary alcohol obtained in c) or the tertiary alcohol obtained in d) is, if desired, reacted with a $C_1$—$C_4$-alkanoyl chloride or with a $C_1$—$C_4$-alkanoyl anhydride in the presence or absence of a solvent or of a diluent and/or of an inorganic or organic base and/or of an acylation catalyst at from 0 to 100°C, or said alcohol or its alkali metal or quaternary ammonium salt is reacted with an alkylating agent of the formula VIII

$$L\text{—}R^4 \qquad \text{VIII}$$

where $R^4$ is $C_1$—$C_4$-alkyl, $C_2$—$C_4$-alkenyl or $C_2$—$C_4$-alkynyl and L is a nucleophilically displaceable leaving group, in the presence or absence of a solvent or of a diluent and/or of inorganic or organic bases and/or of a reaction accelerator at from 0 to 100°C,

e) a Grignard reagent of the formula

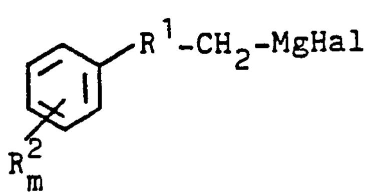

V,

where $R^1$, $R^2$ and m have the meanings stated in claim 1 and Hal is chlorine, bromine or iodine, is reacted with tert-butyl cyanide by a conventional method to give the corresponding ketone VI

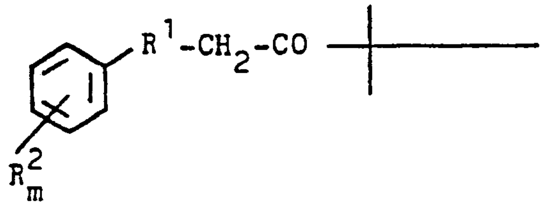

VI

and the latter is halogenated to give a compound of the formula IV and the resulting halide is reacted with imidazole or 1,2,4-triazole in the presence or absence of a solvent or of a diluent and/or of inorganic or organic bases at from 0 to 100°C and, if desired, the procedure is continued according to c) or d),
after which, if desired, the resulting compound of the formula I is converted into its plant-tolerated acid addition salts or metal complexes.